(19)

Europäisches
Patentamt
European
Patent Office
Office européen
des brevets

(11) EP 3 382 610 A1

(12) EUROPEAN PATENT APPLICATION
published in accordance with Art. 153(4) EPC

(43) Date of publication:
03.10.2018 Bulletin 2018/40

(51) Int Cl.:
$G06N\ 99/00$ (2010.01)  $G06F\ 17/15$ (2006.01)
$G06F\ 19/24$ (2011.01)

(21) Application number: 16868513.9

(22) Date of filing: 21.11.2016

(86) International application number:
PCT/JP2016/084509

(87) International publication number:
WO 2017/090566 (01.06.2017 Gazette 2017/22)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 26.11.2015 JP 2015230862

(71) Applicant: Human Metabolome Technologies Inc.
Tsuruoka-shi
Yamagata 9970052 (JP)

(72) Inventor: YAMAMOTO, Hiroyuki
Tsuruoka-shi
Yamagata 997-0052 (JP)

(74) Representative: Prinz & Partner mbB
Patent- und Rechtsanwälte
Rundfunkplatz 2
80335 München (DE)

(54) DATA ANALYSIS APPARATUS, METHOD, AND PROGRAM

(57) A data analysis apparatus performs multivariate analysis with data items on statistical samples. The data analysis apparatus (50) includes a storage (52) and a controller (51). The storage records statistical data (X) for managing the data items per statistical sample, and group information (Y) indicating an arrangement order of groups formed by statistical samples. The controller performs calculation processing based on the statistical data and the group information. The controller calculates, based on the statistical data, a kernel matrix (K) in which a matrix element represents a relationship between a statistical sample corresponding to a row number and a statistical sample corresponding to a column number of the matrix element in the statistical samples. The controller performs calculation processing based on a partial least squares method under a condition defined by the kernel matrix and the group information, to calculate a score for the statistical samples.

FIG. 1A

FIG. 1B

**Description**

TECHNICAL FIELD

The present invention relates to a data analysis apparatus, method, and program which analyze data by a statistical method.

BACKGROUND ART

In the field of statistical data analysis, partial least squares (PLS) method which is one type of the supervised dimension reduction method is used for multivariate analysis such as metabolomics for comprehensively analyzing metabolites in a biological body. The PLS is used for various purposes such as visualization, regression, and construction of discrimination model. Recently, a method obtained by improving PLS is proposed (for example, Patent Document 1).
Patent Document 1 discloses OPLS (orthogonal partial least squares method) in which OSC (orthogonal signal correction) method is applied to the PLS. The OPLS of Patent Document 1 separates, in a model which predicts a variable Y from input data set X by the PLS, a systematic variation in X to a variation orthogonal (uncorrelated) to Y and a variation which enables prediction of Y. Accordingly, variations uncorrelated with Y among variations of multiple statistical samples included in the data set X are filtered. Consequently, it is possible to obtain a model which is more easily interpreted without undermining Y prediction accuracy.

CITATION LIST

PATENT DOCUMENT

Patent Document 1: U.S. Patent Application Publication No. 2003/0,200,040 A

NON-PATENT DOCUMENTS

Non-Patent Document 1: T. Ooga, et al., "Metabolomic anatomy of an animal model revealing homeostatic imbalances in dyslipidaemia", Mol. Biosyst, 7(4).

Non-Patent Document 2: H. Yamamoto, "PLS-ROG: Partial least squares with rank order of groups", COBRA Preprint Series, Working Paper 100, October 2012.

Non-Patent Document 3: C. Urbaniak, et al., "Effect of chemotherapy on the microbiota and metabolome of human milk", a case report, Microbiome, 2014.

Non-Patent Document 4: Lozupone C, et al., "UniFrac: a new phylogenetic method for comparing microbial communities", Appl Environ Microbiol 2005.

SUMMARY OF INVENTION

PROBLEMS TO BE SOLVED BY THE INVENTION

Recently, in the field of metabolomics, there is a case where a plurality of individuals (statistical samples) obtained by sampling data of metabolite are classified into several groups according to blood lines or the status of dosing. In this case, a research related to a variation pattern of the metabolism which variates according to the specific order among the groups is reported (Non-Patent Document 1).
An object of the present invention is to provide a data analysis apparatus, method, and program which can perform various data analysis by taking into account a group order among statistical samples.

MEANS FOR SOLVING THE PROBLEMS

A data analysis apparatus according to the present invention performs multivariate analysis with a plurality of data items on a plurality of statistical samples. The data analysis apparatus includes a storage and a controller. The storage records statistical data for managing the plurality of data items per statistical sample, and group information indicating an arrangement order of groups formed by the plurality of statistical samples. The controller performs predetermined calculation processing based on the statistical data and the group information. The controller calculates, based on the statistical data, a kernel matrix in which a matrix element represents a predetermined relationship between a statistical sample corresponding to a row number of the matrix element in the plurality of statistical samples and a statistical sample corresponding to a column number of the matrix element. The controller performs calculation processing based on a partial least squares method under a predetermined condition defined by the kernel matrix and the group information, to calculate a score for the statistical samples.

EFFECTS OF THE INVENTION

The data analysis apparatus according to the present invention can perform integrated analysis and nonlinear analysis on various types of statistical data based on the kernel matrix while reflecting a group order in scores based on group information. Consequently, it is possible to perform various types of data analysis while taking into account the group order among statistical samples.

BRIEF DESCRIPTION OF DRAWINGS

Fig. 1A and 1B are views for explaining an outline of a data analysis method according to a first embodiment.

Fig. 2 is a chart illustrating metabolome data of a liver sample.

Fig. 3 is a chart illustrating metabolome data of a heart sample.

Fig. 4 is a chart illustrating metabolome data of a brain sample.

Fig. 5 is a chart illustrating metabolome data of a plasma sample.

Fig. 6 is a block diagram illustrating a configuration of a data analysis apparatus according to the first embodiment.

Fig. 7 is a flowchart illustrating data analysis processing by the data analysis apparatus.

Figs. 8A to 8F are expressions for explaining the data analysis processing.

Fig. 9 is a flowchart illustrating kernel PLS-ROG calculation processing of the data analysis processing.

Figs. 10A to 10C are charts for explaining kernel PLS-ROG calculation processing.

Figs. 11A to 11D are charts illustrating analysis data of the data analysis processing.

Figs. 12A and 12B are views illustrating display example of the data analysis processing.

Figs. 13A to 13E are charts for explaining an analysis result of the data analysis processing.

Figs. 14A to 14C are graphs illustrating an analysis result of kernel PLS.

Figs. 15A to 15C are graphs illustrating an analysis result of the kernel PLS-ROG.

EMBODIMENTS OF THE INVENTION

Hereinafter, embodiments of a data analysis apparatus, method, and program according to the present invention will be described with reference to the accompanying drawings. In each following embodiment, the same components will be assigned the same reference numerals.

(First Embodiment)

1. Outline

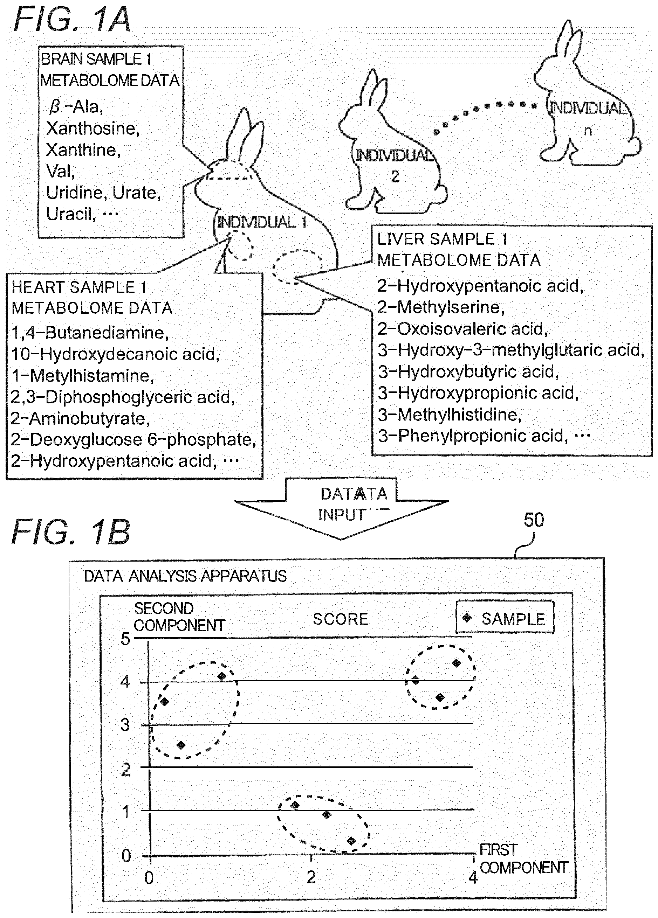

An outline of statistical analysis of a data analysis method according to a first embodiment of the present invention will be described with reference to Figs. 1A to 5. Figs. 1A and 1B are views for explaining the outline of the data analysis method according to the present embodiment. An example of application of this data analysis method to metabolomics will be described below.

The metabolomics is a research field which comprehensively analyzes low molecular metabolites (compounds whose molecular weight is 1000 or less) in a biological body. Fig. 1A illustrates an example in which statistical samples (individuals) to be analyzed are rabbits. In the metabolomics, various analyzing devices measure biological samples (specimens) such as animal tissues, microbial cells, human blood and urine, and analyze the concentration of metabolites contained in each sample. Metabolome data in which values of measured concentrations of various metabolites are recorded is expressed in a following form of a data matrix X of n rows and p columns, for example:

[Math. 1]

$$\mathbf{X} = \begin{bmatrix} x_{11} & x_{12} & x_{13} & \Lambda & x_{1p} \\ M & M & M & O & M \\ x_{n1} & x_{n2} & x_{n3} & \Lambda & x_{np} \end{bmatrix} \quad (1)$$

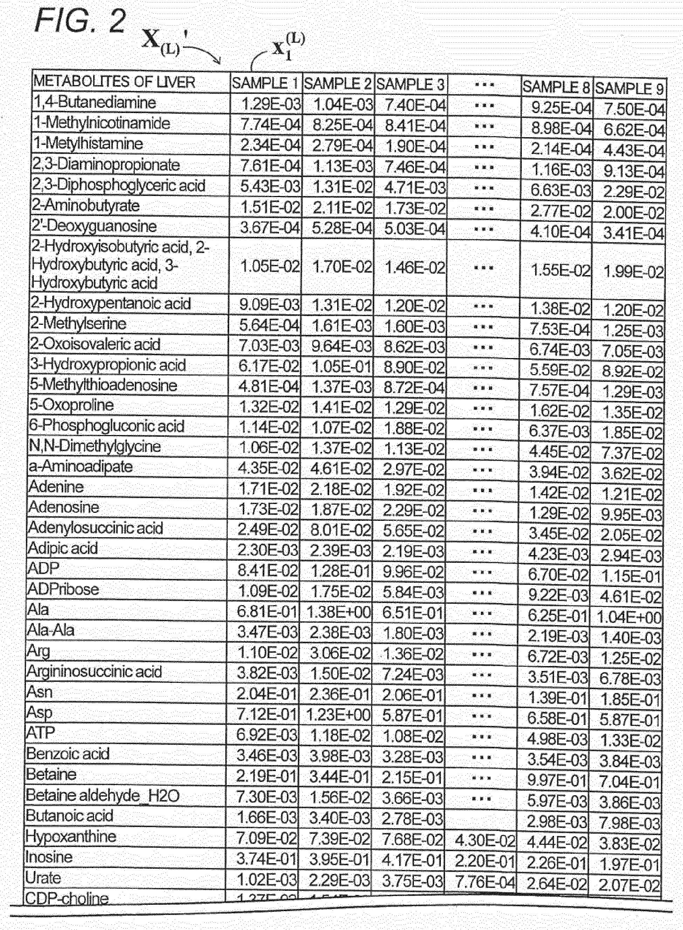

where n represents a sample size (the number of individuals), and p represents the number of measured metabolites (the number of measurement items). According to the formula (1), items of measurement data (statistical data) of p metabolites measured from individuals corresponding to row numbers are recorded per row. Figs. 2 to 5 illustrate examples of the data matrix X.

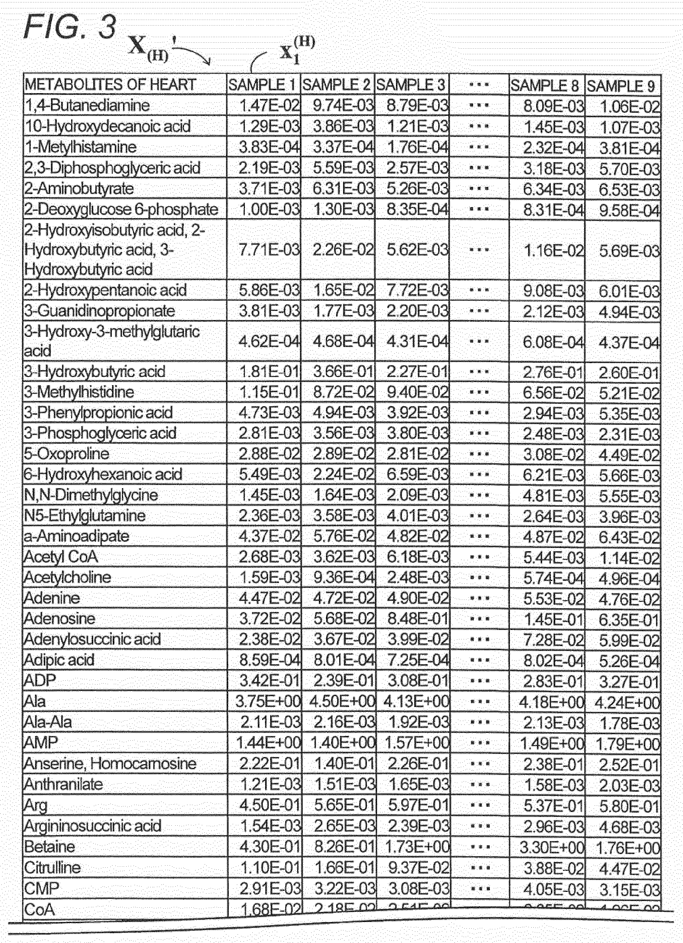

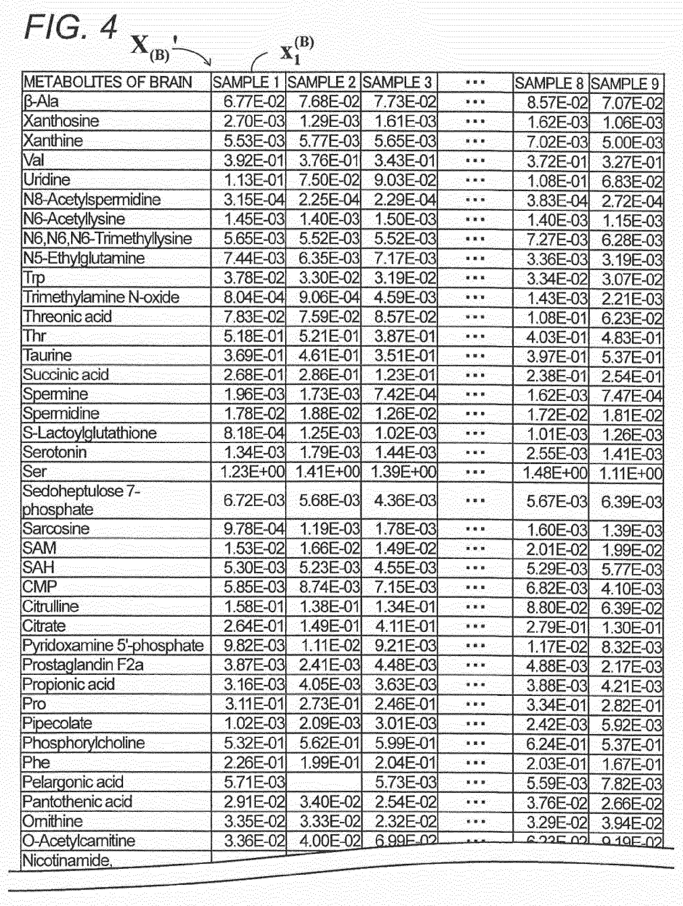

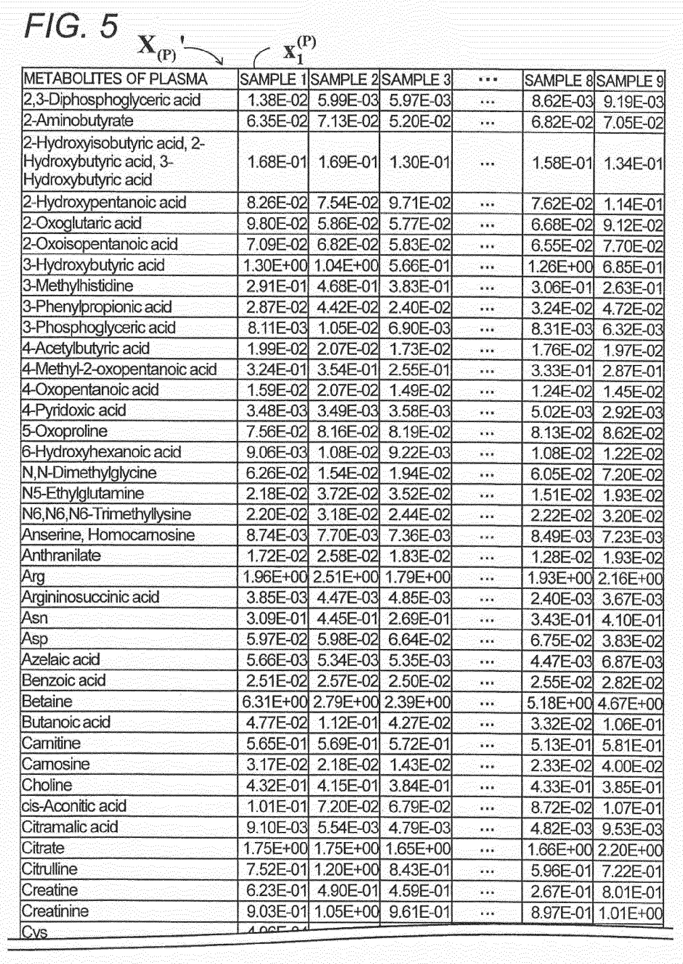

Figs. 2 to 5 are examples of metabolome data obtained by analyzing liver, heart, brain, and plasma samples from nine individual rabbits and measuring metabolites of these samples. Fig. 2 illustrates a data matrix $X_{(L)}$ of the liver samples. Fig. 3 illustrates a data matrix $X_{(H)}$ of the heart samples. Fig. 4 illustrates a data matrix $X_{(B)}$ of the brain samples. Fig. 5 illustrates a data matrix X(p) of the plasma samples. In the drawings,"'" means transposition of the matrix (the same applies hereinafter).

The metabolome data illustrated in Figs. 2 to 5 of each of the liver, heart, brain, and plasma samples of the nine individuals are measured by using a capillary electrophoresis-time of flight mass spectrometer. For example, the data in a column of a "sample 1" in Fig. 2 indicates measurement data $x_1^{(L)}$ obtained by measuring liver metabolites of the first rabbit of the nine individuals. Although not illustrated, the numbers of metabolites detected from each biological sample are 170 in the liver, 161 in the heart, 159 in the brain, and 129 in the plasma, respectively.

As in the above example, the metabolome data contains measurement data of several hundreds to several thousands of metabolites. These complicate to visually express the behavior of each sample on the metabolome data (for example, in a case that normal mice and disease model mice are analysis targets, what difference the metabolome data of those liver samples causes). Thus, scores based on a multivariate are generated by multivariate analysis to visually express the behavior of samples by using a scatter plot of scores. The scatter plot is used to check a relevance between samples (such as a difference between two groups of the normal mice and the disease model mice) as illustrated in Fig. 1B.

Here, in the examples illustrated in Figs. 1 to 5, individuals of first to third sample numbers are wild rabbits, and fourth to ninth individuals are WHHL rabbits (hyperlipemia model rabbits). Among the fourth to ninth rabbits, statin is administered to the third to sixth WHHL rabbits and the statin is not administered to the seventh to ninth WHHL rabbits. Therefore, in these examples, there are three groups consisting of the first to third individuals, the fourth to sixth individuals, and the seventh to ninth individuals.

In the above case, obtaining such scores that three groups are arranged in a predetermined order is useful for biological observation related to the order and its verification. With respect to the metabolome data obtained from each individual, which is managed per type of biological sample as illustrated in Figs. 2 to 5, integral analysis of these items of data is required in some cases. In view of the above demands, the present invention provides the data analysis method which enables various data analysis while taking into account a group order among individuals. The theory of the data analysis method according to the present embodiment will be described below.

2. Theory

First, a general theory on multivariate analysis of metabolomics will be described. The multivariate analysis of metabolome data generally uses main component analysis and PLS. The PLS can easily obtain accurate scores whose groups are precisely separated by using group information in combination with metabolome data additionally. A classical multivariate analyzing method for performing analysis by using group information includes, for example, a canonical correlation analysis. However, it is difficult to apply this method to data when the number (p) of variables (measurement items) in the data is larger than a sample size (n) (p >> n). In contrast to this, PLS is also applicable in the case of p >> n.

By using PLS, it is possible to obtain scores whose groups are separated. In this regard, a predetermined order among groups is assumed in some case, for example, where variations related to a drug concentration is of interest, or attention is paid to metabolites related to tastiness indices in sensory evaluation. However, the PLS does not reflect the group order information in the scores, and thus cannot obtain expected results. Therefore, the inventor proposed a method called PLS-ROG (Rank Order of Groups) to which PLS is modified (see Non-Patent Document 2). Using PLS-ROG makes it possible to obtain scores whose groups have an order. Further, the PLS-ROG enables selection of metabolites related to scores by using statistical hypothesis testing.

In the metabolomics, multiple types of metabolome data may be obtained from one individual. For example, administration of a particular drug to an animal is likely to influence metabolism of multiple organs. In such a case, samples (specimens) of a plurality of organs, plasma, and urine are sampled from the identical individual, and respective metabolome data are obtained. In addition, data other than the metabolome data, such as a gene expression level and a protein level, is often measured simultaneously with metabolome data from the identical individual. By integrating plural measurement data obtained from the identical individual and calculating common scores by using multivariate analysis, it is possible to specify metabolites which commonly variate in a plurality of organs, or metabolites and genes which variate commonly in the identical individual.

If the above multivariate analysis can integrate different types of measurement data with the group order among individuals reflected, it is possible to perform various data analysis, e.g., possible to specify metabolites which commonly variate in individuals according to the group order, or causal relationships of them. Therefore, the inventor devises "kernel PLS-ROG (kernel order partial least squares method)" by introducing a concept of a kernel method to the above PLS-ROG, the kernel PLS-ROG enabling various analysis such as integrated analysis of various types of measurement data and nonlinear data analysis while taking into account the order among the groups. Hereinafter, the PLS-ROG and the kernel PLS-ROG will be described.

2-1. PLS-ROG

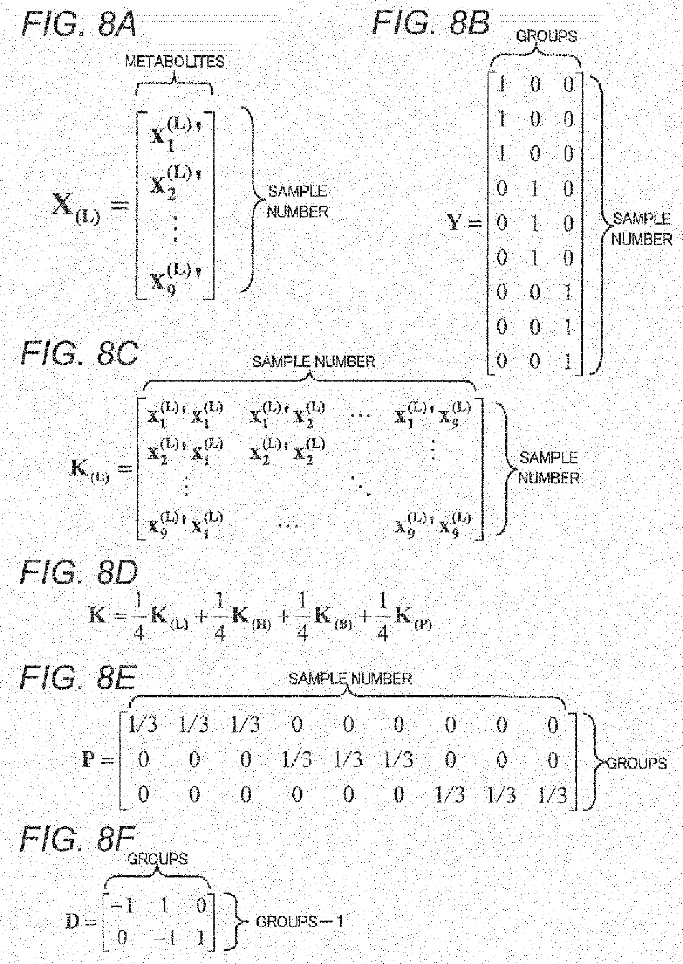

The PLS-ROG can be formulated by using the data matrix X (formula (1)) having n rows and p columns, a dummy matrix Y having n rows and g columns, and an explanatory variable t and an objective variable s (each n-dimensional vector). Here, n represents a sample size, p represents the number of measurement items (data items), and g represents the number of groups. The dummy matrix Y is a matrix for setting group information indicating the group order (see Fig. 8B). The explanatory variable t and the objective variable s constitute a synthetic variable (t, s).

Following relationships are set between the explanatory variable t and the data matrix X by using a weight vector $w_x$ (p-dimensional vector), and between the objective variable s and the dummy matrix Y to set the following relationships by using a weight vector $w_y$ (g-dimensional vector) respectively.

$$t = Xw_x \quad (2)$$

$$s = Yw_y \quad (3)$$

By using the above X, Y, t and s, the PLS-ROG is formulated as the following optimization problem (to find specific weight vectors $w_x$, and $w_y$).

[Math. 2]

$$\max \mathrm{cov}(t, s) \qquad (4)$$

$$subject \quad to \quad \mathbf{w}_x{}' \mathbf{w}_x = 1, \qquad (5)$$

$$(1-\kappa)\mathbf{w}_y{}'\mathbf{w}_y + \kappa\mathbf{w}_y{}'\mathbf{Y}'\mathbf{P}'\mathbf{D}'\mathbf{D}\mathbf{P}\mathbf{Y}\mathbf{w}_y = 1 \qquad (6)$$

In the above formula, cov(t, s) is a covariance of the explanatory variable t and the objective variable s, and $\kappa$ is a parameter constant indicating a penalty for the group order among individuals. The matrix P is a matrix of g rows and n columns indicating weights corresponding to the number of individuals (number of samples) $n_1$, $n_2$, ..., and $n_g$ included in each group. The matrix D is a matrix of (g-1) rows and g columns for smoothing among groups. Concrete forms of the matrices P and D is exemplified as below.
[Math. 3]

$$\mathbf{P} = \begin{bmatrix} 1/n_1 & \Lambda & 1/n_1 & \mathbf{0} & & \mathbf{0} \\ \mathbf{0} & & & O & & \mathbf{0} \\ \mathbf{0} & & & \mathbf{0} & 1/n_g & \Lambda & 1/n_g \end{bmatrix} \qquad (7)$$

$$\mathbf{D} = \begin{bmatrix} -1 & 1 & 0 & \Lambda & 0 & 0 \\ 0 & -1 & 1 & \Lambda & 0 & 0 \\ M & M & M & O & M & M \\ 0 & 0 & 0 & \Lambda & -1 & 1 \end{bmatrix} \qquad (8)$$

According to the formulas (4) to (6), the PLS-ROG configures an optimization problem for optimizing the covariance cov (t, s) under the conditions expressed by the formulas (5) and (6). The conditional formula (5) expresses a condition for setting a magnitude of the weight vector $w_x$ to 1. The conditional formula (6) expresses a condition for shifting the magnitude of the weight vector $w_y$ from 1 by the constant $\kappa$ according to a penalty term as the second term on the left side. The second term on the left side of the formula (6) is the penalty term which gives a penalty according to the group order of the dummy matrix Y.
The score according to the PLS-ROG is calculated by $w_x$ and $w_y$ obtained in the optimization problem and a corresponding synthetic variable (t, s) in the formulas (2) and (3). The PLS-ROG can reflect the group order set by the dummy matrix Y in the scores according to the penalty term of the conditional formula (6).

2-2. Kernel PLS-ROG

The kernel PLS-ROG, which is a statistical data analyzing method according to the present embodiment, will be described below.

2-2-1. Formulation of Kernel PLS-ROG

First, the formulation of the kernel PLS-ROG will be described. In the formulas (2) to (6) obtained by formulating the PLS-ROG, the following formula (9) is adopted instead of the formula (2). Along with this, a kernel matrix K having n rows and n columns and an n-dimensional vector $\alpha_x$ are introduced (formulas (10) and (11)).

$$t = \Phi w_x \qquad (9)$$

$$w_x = \Phi' \alpha_x \qquad (10)$$

$$K = \Phi\Phi' \qquad (11)$$

In the above formula, $\Phi$ represents a matrix (mapping) corresponding to the data matrix X. A specific matrix indication (n rows and p columns) of $\Phi$ may not be given in particular. The kernel matrix K is a matrix in which each of matrix elements are composed of a kernel function $k(x_i, x_j)$ taking, as arguments, two of the plural measurement data $x_i$ (p-dimensional vectors) for every sample in the data matrix X. The kernel function $k(x_i, x_j)$ is a function which represents an inner product in the feature space to which $x_i$, $x_j$ are mapped by $\Phi$, and has a concrete form which can be calculated based on a pair of measurement data $x_i$ and $x_j$. Details of the kernel matrix K and the kernel function $k(x_i, x_j)$ will be described later. The vector $\alpha_x$ is a vector used in place of the weight vector $w_x$.

With the formulas (9) to (11), the explanatory variable t can be expressed as the following formula by using the vector $\alpha_x$ and the kernel matrix K without using $w_x$ and $\Phi$.

$$t = K\alpha_x \qquad (12)$$

Further, the formula (5) is expressed as the following formula by using the vector $\alpha_x$ and the kernel matrix K based on the formula (10).

$$\alpha_x'K\alpha_x = 1 \qquad (13)$$

The formula (13) expresses a condition that an inner product via the kernel matrix K between the vector $\alpha_x$ and itself is 1. As a result, the kernel PLS-ROG configures the optimization problem in which, instead of the formula (5), the condition of the formula (13) is imposed on the formulas (4) to (6) for formulating the PLS-ROG. The kernel PLS-ROG is described by the formulas (4), (6), and (13) without using the concrete form of $\Phi$ by eliminating the weight vector $w_x$.

The kernel PLS-ROG formulated as the above can be described as the optimization problem of the following Lagrangian function J ($\lambda_x$ and $\lambda_y$ represent parameters) by using the Lagrangian multiplier method.

[Math. 4]

$$J = \frac{1}{n-1}\boldsymbol{\alpha_x}'\mathbf{KYw_y} + \lambda_x(1 - \boldsymbol{\alpha_x}'\mathbf{K}\boldsymbol{\alpha_x})$$
$$+ \lambda_y\left\{1 - (1-\kappa)\mathbf{w_y}'\mathbf{w_y} - \kappa\mathbf{w_y}'\mathbf{Y'P'D'DPYw_y}\right\} \qquad (14)$$

By partially differentiating the function J with $\alpha_x$ and $w_y$ respectively and rearranging the two obtained formulas, the kernel PLS-ROG finally returns to the generalized eigenvalue problem of the following formula (calculation of eigenvalue $\lambda$ and eigenvectors $\alpha_x$ and $w_y$).

[Math. 5]

$$\frac{1}{(n-1)^2}\mathbf{KY}\{(1-\kappa)\mathbf{I} + \kappa\mathbf{Y'P'D'DPY}\}^{-1}\mathbf{Y'K}\boldsymbol{\alpha_x} = \lambda\mathbf{K}\boldsymbol{\alpha_x} \qquad (15)$$

$$\frac{1}{(n-1)^2}\mathbf{Y'KYw}_y = \lambda\{(1-\kappa)\mathbf{I} + \kappa\mathbf{Y'P'D'DPY}\}\mathbf{w}_y \qquad (16)$$

The eigenvalues $\lambda$ and the eigenvectors $\alpha_x$ and $w_y$ calculated according to the formulas (15) and (16) have (g-1) non-zero eigenvalues $\lambda$. In the present embodiment, the score is set to a value of the explanatory variable t obtained by substituting the eigenvector $\alpha_x$ of each eigenvalue $\lambda$ in the formula (12).

The formulas (15) and (16) are used by a data analysis apparatus 50 (see Fig. 1B) according to the present embodiment as arithmetic expressions for calculating the kernel PLS-ROG (the PLS under the conditions of the formulas (6) and (13)). The data analysis apparatus 50 will be described later.

2-2-2. Kernel Matrix

Details of the kernel matrix and the kernel function will be described below.

The (i, j) element of the kernel matrix K is expressed as the kernel function $k(x_i, x_j)$ related to the pair of the

measurement data $x_i$ and $x_j$ which are for the ith and jth samples in the data matrix X. Various concrete forms of the kernel function $k(x_i, x_j)$ can be used. For example, the following linear kernel $k_L(x_i, x_j)$ (formula (17)), a polynomial kernel $k_P(x_i, x_j)$ (formula (18)), and a Gaussian kernel $k_G(x_i, x_j)$ (formula (19)) can be used as the kernel function $k(x_i, x_j)$.

[Math. 6]

$$k_L(\mathbf{x_i}, \mathbf{x_j}) = \mathbf{x_i}'\mathbf{x_j} \qquad (17)$$

$$k_P(\mathbf{x_i}, \mathbf{x_j}) = (\mathbf{x_i}'\mathbf{x_j} + m)^q \qquad (18)$$

$$k_G(\mathbf{x_i}, \mathbf{x_j}) = \exp(-\frac{\left\|\mathbf{x_i} - \mathbf{x_j}\right\|^2}{\sigma^2}) \qquad (19)$$

In the formula (18), m represents an arbitrary real number, q represents an arbitrary natural number, and σ in the formula (19) represents a positive real number. By forming the kernel matrix K based on the nonlinear kernels such as the formulas (18) and (19), it is possible to perform nonlinear data analysis while taking into account the group order.

Further, when a plurality of types of measurement data $x_i^{(L)}$, $x_i^{(H)}$, $x_i^{(B)}$, and $x_i^{(P)}$ is obtained per individual as in the case of metabolome data derived from a plurality of organs or biological body fluids (see Figs. 2 to 5), the kernel matrix K used for integrated analysis of measurement data managed per type can be configured as follows.

Assuming N types of measurement data are obtained per individual, measurement items per type are recorded in each type of data matrix $X_{(1)}$, $X_{(2)}$, ..., and $x_{(N)}$, and the column directions do not coincide with each other. In this case, calculating the kernel matrix based on the kernel function for each of the various types of measurement data $x_i^{(1)}$, $x_i^{(2)}$, ..., and $x_i^{(N)}$, result in that the kernel matrices $K_{(1)}$, $K_{(2)}$, ..., and $K_{(N)}$ all have n rows and n columns per type. The kernel matrix K for integrated analysis is composed of a predetermined average of all types of kernel matrices $K_{(1)}$, $K_{(2)}$, ..., and $K_{(N)}$. The predetermined average may be an arithmetic mean, a weighted mean with an appropriately selected weight, or a geometric mean per each matrix element.

The theory of the kernel PLS-ROG configured as described above can be realized by a computer for calculating the kernel matrix K based on the data matrix X indicating measurement data of a plurality of statistical samples, and calculating the formulas (15) and (16) based on the kernel matrix K and group information related to a group order among statistical samples. In this way, it is possible to obtain scores which take into account the group order among statistical samples on the computer, so as to visualize the scores by plot display, and integrally analyze a plurality of types of data matrices $X_{(1)}$, $X_{(2)}$, ..., and $X_{(N)}$. Hereinafter, the data analysis apparatus, method, and program which realize the kernel PLS-ROG will be described.

3. Data Analysis Apparatus, Method, and Program

3-1. Configuration

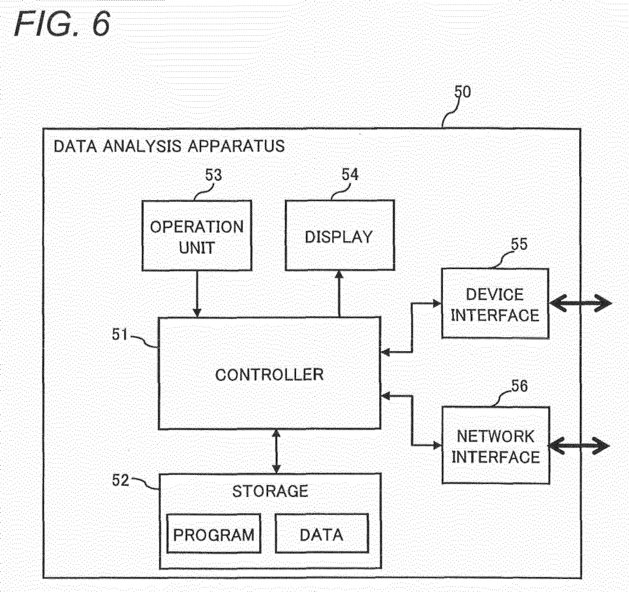

The configuration of the data analysis apparatus 50 according to the present embodiment will be described with reference to Fig. 6. Fig. 6 is a block diagram illustrating the configuration of the data analysis apparatus 50.

The data analysis apparatus 50 performs calculation according to the kernel PLS-ROG (PLS under the conditions of the formulas (6) and (13)) based on the data matrix X indicating the measurement data of a plurality of statistical samples to calculate scores (t), and displays a plot image of the scores (see Fig. 1B). The data analysis apparatus 50 is configured by an information processing device such as a PC (personal computer), for example. As illustrated in Fig. 6, the data analysis apparatus 50 includes a controller 51, a storage 52, an operation unit 53, a display 54, a device interface 55, and a network interface 56.

The controller 51 is configured by, for example, a CPU or an MPU which realizes predetermined functions in cooperation with software, and controls an entire operation of the data analysis apparatus 50. The controller 51 reads data and programs stored in the storage 52 to perform various calculation processing, and realize various functions. For example, the controller 51 executes data analysis processing which realizes data analysis according to the above-described kernel PLS-ROG. The program for executing the data analysis processing may be packaged software. The controller 51 may be a hardware circuit such as a dedicated electronic circuit designed to realize a predetermined function or a reconfigurable electronic circuit. The controller 51 may be configured by various semiconductor integrated circuits such as a CPU, a MPU, a microcomputer, a DSP, a FPGA, and an ASIC.

The storage 52 is a storage medium which stores necessary programs and data to realize the functions of the data analysis apparatus 50, and includes, for example, a hard disk (HDD) or a semiconductor storage device (SSD). The storage 52 may include, for example, a semiconductor device such as a DRAM or an SRAM, to temporarily store data and function as a working area of the controller 51. For example, the storage 52 stores arithmetic expressions (formulas (15) and (16)) of the kernel PLS-ROG, the data matrix X (indicating measurement data of a plurality of measurement items per statistical sample), the dummy matrix Y (indicating group information related to a group order among statistical samples), and the kernel matrix K. When N types of measurement data are obtained per statistical sample for the data matrix X, the storage 52 manages a plurality of types of data matrices $x_{(1)}$, $x_{(2)}$, ..., and $x_{(N)}$.

The operation unit 53 is a user interface through which a user performs an operation. The operation unit 53 is configured by, for example, a keyboard, a touch pad, a touch panel, buttons, switches, and combinations thereof. The operation unit 53 is an exemplary obtaining unit which obtains various information input by the user.

The display 54 is configured by, for example, a liquid crystal display or an organic EL display. The display 54 displays various information such as information input from the operation unit 53, for example.

The device interface 55 is a circuit (module) which connects other devices to the data analysis apparatus 50. The device interface 55 performs communication according to a predetermined communication standard. The predetermined standards include USB, HDMI (registered trademark), IEEE 1395, WiFi, and Bluetooth (registered trademark).

The network interface 56 is a circuit (module) which connects the data analysis apparatus 50 to the network via a wireless or wired communication line. The network interface 56 performs communication conforming to predetermined communication standards. The predetermined communication standards include communication standards such as IEEE802.3, and IEEE802.11a/11b/11g/11ac.

3-2. Operation

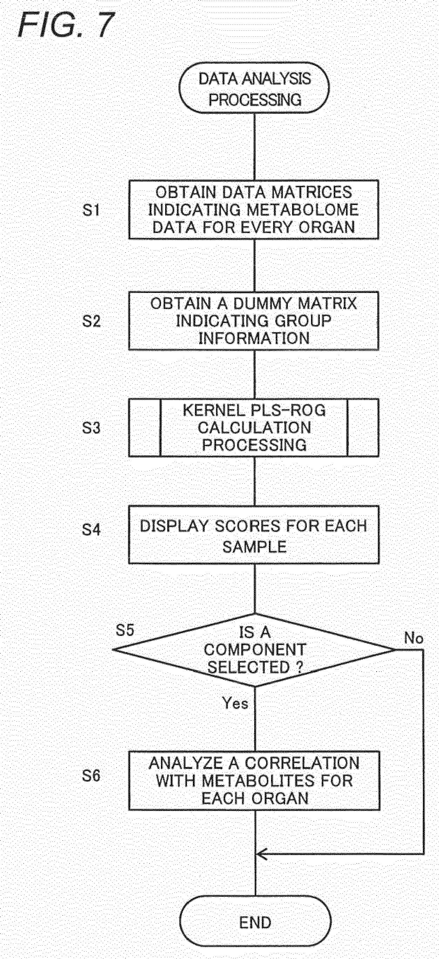

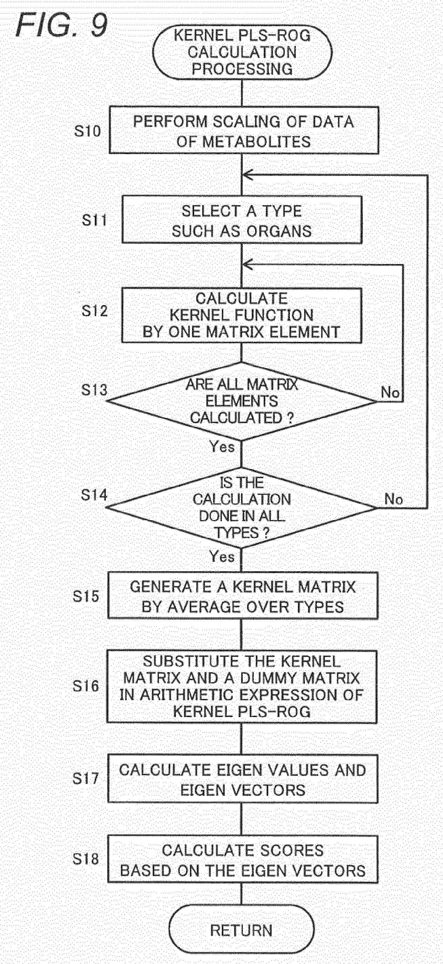

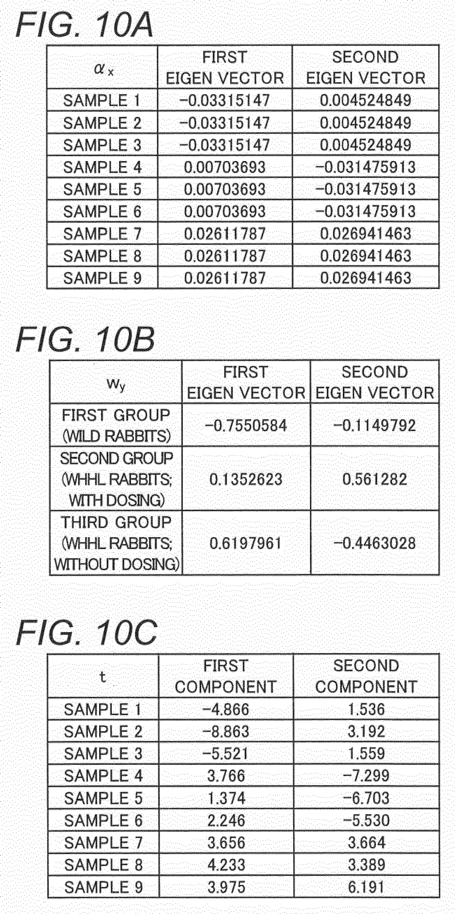

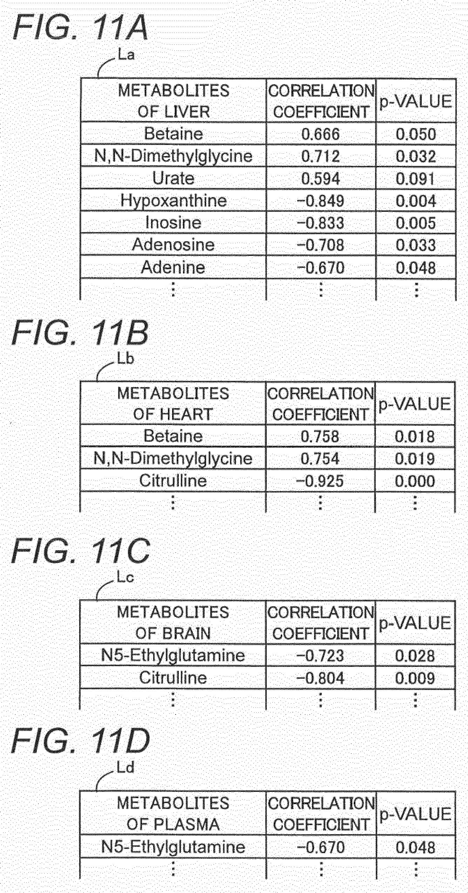

The operation of the data analysis apparatus 50 according to the present embodiment will be described with reference to Figs. 7 to 11. Fig. 7 is a flowchart illustrating the data analysis processing by the data analysis apparatus 50. Figs. 8A to 8F are expressions for explaining the data analysis processing. Fig. 9 is a flowchart illustrating the kernel PLS-ROG calculation processing in the data analysis processing. Figs. 10A to 10C are charts for explaining the kernel PLS-ROG calculation processing. Figs. 11A to 11D are charts illustrating analysis data of data analysis processing.

The flowcharts illustrated in Figs. 7 and 9 are executed by the controller 51 of the data analysis apparatus 50. Hereinafter, an operation example of the data analysis apparatus 50 will be described, in which the nine individual rabbits form three groups, and metabolome data of each of four types of biological samples such as the liver, the heart, the brain, and the plasma is obtained from each individual (see Figs. 1 to 5).

In the following operation example, the storage 52 stores in advance the various data matrices $x_{(L)}$, $x_{(H)}$, $x_{(B)}$, and $X_{(P)}$ indicating the metabolome data of each type of the liver, the heart, the brain, and the plasma (see Figs. 2 to 5), and the dummy matrix Y indicating group information related to the group order among individuals.

In the flowchart of Fig. 7, at first, the controller 51 obtains the various data matrices $x_{(L)}$, $x_{(H)}$, $x_{(B)}$, and $x_{(P)}$ from the storage 52 (S1). Fig. 8A illustrates an example of the various data matrices $x_{(L)}$. As illustrated in Fig. 8A, each row of the data matrix $X_{(L)}$ records measurement data $x^{(L)}_i$ obtained by measuring metabolites of a specific biological sample of each individual per row.

Returning to Fig. 7, the controller 51 then obtains the dummy matrix Y from the storage 52 (S2). The dummy matrix Y is set when, for example, the user inputs metabolome data. Fig. 8B illustrates an example of the dummy matrix Y. In the dummy matrix illustrated in Fig. 8B, the first to third rabbits of the nine individuals form the first group (first column), the fourth to sixth rabbits form the second group (second column), and the seventh to ninth rabbits form the third group (third column).

Next, the controller 51 performs the kernel PLS-ROG calculation processing based on the obtained data matrices $x_{(L)}$, $x_{(H)}$, $x_{(B)}$, and $x_{(P)}$ and dummy matrix Y (S3). The kernel PLS-ROG calculation processing is processing for calculating the formulas (15) and (16) of the kernel PLS-ROG described in the above 2-2-2. This processing will be described with reference to Figs. 8C to 8F.

Here, the kernel PLS-ROG calculation processing (S3) will be described with reference to the flowchart of Fig. 9. At first, the controller 51 performs scaling (normalization) of data in the data matrices $x_{(L)}$, $x_{(H)}$, $x_{(B)}$, and $x_{(P)}$ of each organ and plasma such that the average among individuals are 0 and the variance is 1 for each metabolite (S10).

Next, the controller 51 selects one type of a plurality of types of data matrices $x_{(L)}$, $x_{(H)}$, $x_{(B)}$, and $x_{(P)}$ (e.g. a liver sample) (S11).

Next, based on the measurement data (of the liver sample) $x_i^{(L)}$ and $x_j^{(L)}$ (i, j = 1 to 9) for a pair of individuals among all nine individuals, the controller 51 calculates the kernel function $k(x_i^{(L)}, x_j^{(L)})$ as the (i, j) element of the kernel matrix $K_{(L)}$ of the selected type (S12).

The controller 51 performs the calculation of step S12 on all pairs of combinations of all nine individuals, to calculate every matrix element of the kernel matrix $K_{(L)}$ per type (S13). For example, in the case of linear kernel, the controller 51 calculates the matrix elements of the kernel matrix $K_{(L)}$ based on the inner product of a pair of measurement data $x_i^{(L)}$ and $x_j^{(L)}$ as illustrated in Fig. 8C.

The controller 51 performs the processing of steps S11 to S13 on each type of the data matrix $x_{(L)}$, $x_{(H)}$, $x_{(B)}$, and $x_{(P)}$ (S14), to calculate all types of kernel matrices $K_{(L)}$, $K_{(H)}$, $K_{(B)}$, and $K_{(P)}$.

When calculating all types of the kernel matrices $x_{(L)}$, $x_{(H)}$, $x_{(B)}$, and $X(p)$ (Yes in S14), the controller 51 takes an average of types according to the arithmetic expression illustrated in Fig. 8D, to calculate the kernel matrix K (S15).

Next, the controller 51 reads, from the storage 52, the arithmetic expressions (15) and (16) in the theory of the kernel PLS-ROG described in the above section 2-2-2, and substitutes the averaged kernel matrix K and dummy matrix Y in the arithmetic expression (S16). Figs. 8E and 8F illustrate matrices P and D in this example. Penalty terms in the kernel PLS-ROG are calculated based on the matrices Y, D, and P in Figs. 8B, 8E, and 8F.

Next, the controller 51 calculates the eigenvalue $\lambda$ of the generalized eigenvalue problem with the substituted arithmetic expression, and the eigenvectors $\alpha_x$ and $w_y$ corresponding to each eigenvalue $\lambda$ (S17). Figs. 10A and 10B illustrate examples of the eigenvectors $\alpha_x$ and $w_y$ calculated in step S17. In this example (g = 3), two eigenvectors are calculated in accordance with the (g-1) eigenvalues $\lambda$ as illustrated in Figs. 10A and 10B.

Next, the controller 51 calculates the explanatory variable t (n = 9-dimensional vector) corresponding to each of the calculated (g-1) eigenvectors based on the calculated kernel matrix K (formula (12)), to calculate the scores for each individual (S18). Fig. 10C illustrates an example of the scores calculated in step S18. The explanatory variable t is an n (= 9) dimensional vector, and each vector element is a score for each individual. The score of each individual has first to (g-1)th components according to the (g-1) eigenvectors. The scores illustrated in Fig. 10C has a first component and a second component according to g = 3.

Returning to Fig. 7, after performing the kernel PLS-ROG calculation processing as described above, as illustrated in Fig. 1B, the controller 51 displays plots of the scores for each sample on the display 54 based on the calculated scores (S4).

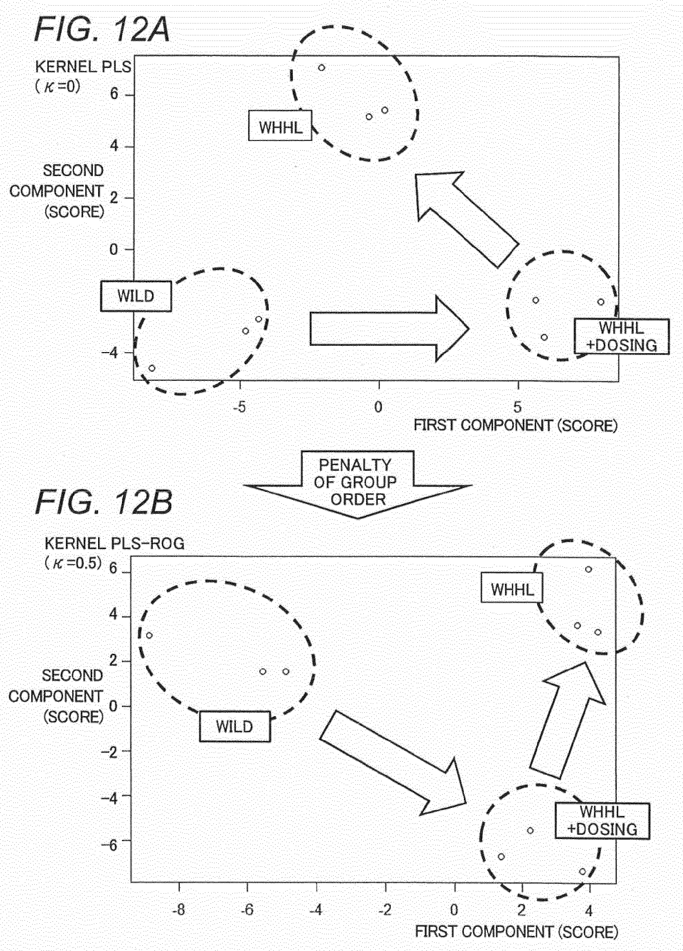

Next, the controller 51 receives a user's operation via the operation unit 53, and determines whether or not the user selects any one of the components of the displayed scores for further data analysis (S5). For example, the user can select the score component which reflects the group order from a plot image of the score displayed on the display 54 (see Fig. 12B).

When it is determined that the user does not select any score component (No in S5), the controller 51 ends the present processing.

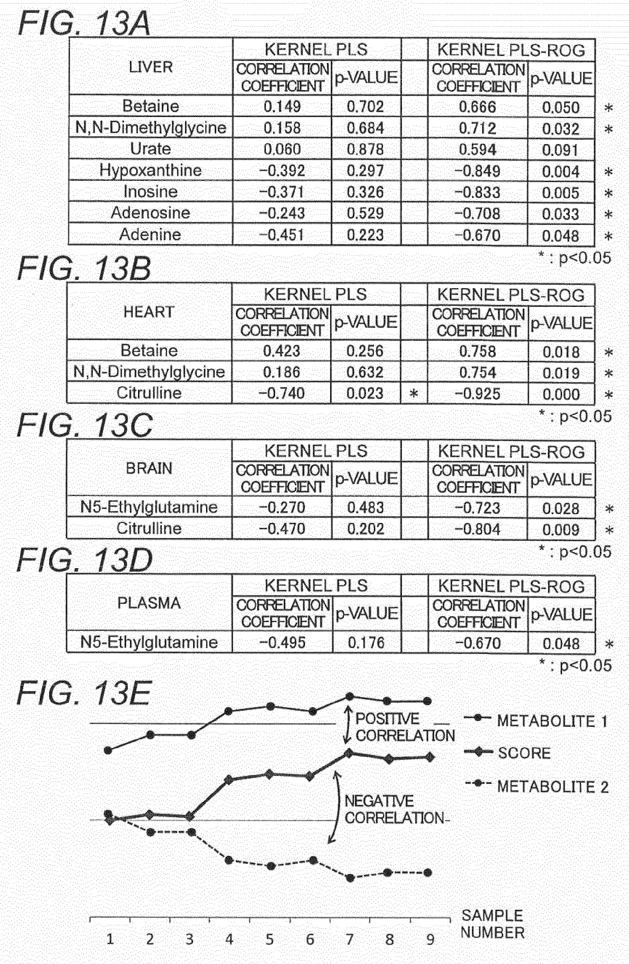

On the other hand, when it is determined that the user selects any one of the score components (Yes in S5), the controller 51 analyzes a correlation between metabolites of each of the various data matrices $x_{(L)}$, $x_{(H)}$, $x_{(B)}$, and $x_{(P)}$ and the score of the selected component (S6). More specifically, the controller 51 calculates a correlation coefficient (a coefficient indicating the correlation between statistical distributions of both data (see Fig. 13E) between metabolite data of all individuals and selected component data, and the p value (a probability that a correlation between data occurs by chance), generates a list of the calculation result, and ends this processing.

Figs. 11A to 11D illustrate examples of an analysis result in step S6. Fig. 11A illustrates an analysis list La of metabolites in the data matrix $X_{(L)}$ of the liver sample. Fig. 11B illustrates an analysis list Lb of metabolites in the data matrix $x_{(H)}$ of the heart sample. Fig. 11C illustrates an analysis list Lc of metabolites in the data matrix $x_{(B)}$ of the brain sample. Fig. 11D illustrates an analysis list Ld of metabolites in the data matrix $x_{(P)}$ of the plasma sample.

The analysis lists La to Ld illustrated in Figs. 11A to 11D show correlations between the first component of the score in the case of $\kappa = 0.5$ and the metabolites in the four types of the data matrices $X_{(L)}$ to $x_{(P)}$. In the analysis lists La to Ld, the "correlation coefficient" and the "p value" calculated per metabolite are recorded. According to the analysis lists La to Ld, it is possible to integrally analyze the correlation with a common score for each metabolite of the liver, heart, brain, and plasma samples. Details of the analysis result will be described later.

According to the above data analysis processing, it is possible to realize the kernel PLS-ROG which enables integrated analysis among types according to the kernel matrix K while taking into account the group order among individuals. Hereinafter, the analysis result of the data analysis processing will be described.

3-3. Analysis Result

A biological research (Non-Patent Document 1) suggests that the concentrations of metabolites of metabolic intermediates (N, N-Dimethylglycine and Betaine) and purine metabolism in a glycine biosynthesis pathway of the liver increase/decrease in the order of the wild rabbits (first group), the WHHL rabbits with dosing (second group), and the WHHL rabbits (third group). From this point of view, the data analysis processing (Fig. 7) is performed with the order of the first, second, and third groups in this example set by the dummy matrix Y as shown in Fig. 8B.

Figs. 12A and 12B illustrate display examples ($\kappa$ = 0 or 0.5) in step S4 of the data analysis processing (Fig. 7). The plots in Figs. 12A and 12B illustrate scores of the three individuals of the wild rabbits, the three individuals of the WHHL rabbits with dosing, and the three individuals of the WHHL rabbits. In Figs. 12A and 12B, horizontal axes indicate the first component of the score and vertical axes indicate the second component.

Fig. 12A is a display example of scores obtained in a state (kernel PLS) where there is no penalty term based on the matrices D, P, and Y in the formula (6). In the display example of Fig. 12A, the score of each sample of the three groups is not in the order of the first group (wild rabbits), the second group (WHHL rabbits with dosing), and the third group (WHHL rabbits) as set in the dummy matrix Y (Fig. 8B) in both the first component and the second component.

Fig. 12B is a display example of scores obtained in a state (kernel PLS-ROG) where there is a penalty term in the formula (6). In the display example of Fig. 12B, the scores of each individual of the three groups increase in the order of the first group (wild rabbits), the second group (WHHL rabbits with dosing), and the third group (WHHL rabbits) as set in the dummy matrix Y in the first component. Thus, according to the data analysis processing for realizing the kernel PLS-ROG, the group order can be reflected in the scores according to the penalty term based on the matrix Y.

Further, in the data analysis processing (Fig. 7), various types of metabolome data of the liver, heart, brain, and plasma samples are further analyzed for the score of the first component which reflects the group order. More specifically, the correlation coefficient between the score of the first component and each metabolite, and the p value are calculated (step S6 in Fig. 7) to perform hypothesis testing on each correlation and determine significant (positive in terms of hypothesis testing) metabolites.

Figs. 13A to 13E are charts for explaining hypothesis testing based on processing of step S6 of the data analysis processing (Fig. 7). The tables in Figs. 13A, 13B, 13C, and 13D respectively illustrate the analysis lists La, Lb, Lc, and Ld of the liver, heart, brain, and plasma samples (Fig. 11) obtained in step S6 of Fig. 7.

Fig. 13E is a chart for explaining a correlation coefficient between scores and metabolite data. As illustrated in Fig. 13E, scores and data for various metabolites are distributed throughout the nine individuals. The correlation coefficient expresses the similarity of a distribution of the both data as values within a range of -1 to +1. As the similarity between the scores and the metabolite data is smaller, the correlation coefficient is closer to "0", and is considered to be uncorrelated. Also, as the degree of similarity is larger, an absolute value of the correlation coefficient becomes closer to "1". When the correlation coefficient is closer to "+1", a positive correlation is expected. When the correlation coefficient is closer to "-1", a negative correlation is expected.

Further, in order to determine stochastically whether the above correlation can actually have a meaning (significant) or is a mere coincidence on the data, the p value is used in the hypothesis testing. In this analysis, as illustrated in Figs. 13A to 13D, the significance of the correlation between the score and metabolite data is determined by using "0.05" of the threshold value of the p value.

In the tables of 13A to 13D, information indicated by the analysis lists La to Ld is illustrated together in the case of the kernel PLS (Fig. 12A) and in the case of the kernel PLS-ROG (Fig. 12B). In addition, in Figs. 13A to 13D, "*" is assigned to metabolites whose correlation with the score is confirmed by the hypothesis testing.

As illustrated in Figs. 13A and 13B, all correlation coefficients of the score of the first component of the kernel PLS-ROG in Betaine and N, N-Dimethylglycine (metabolic intermediates of the glycine biosynthetic pathway) of the liver sample and the heart sample are 0.6 or more. In addition, these p values are 0.05 or less, and are positively correlated significantly (to such a degree that this is not by chance stochastically).

Further, regarding the purine metabolism, as illustrated in Fig. 13A, both of the p values of the kernel PLS and the kernel PLS-ROG are 0.05 or more in Urate (uric acid), and do not have a significant correlation. However, the correlation coefficient "0.594" of the kernel PLS-ROG substantially improves from the correlation coefficient "0.0060" of the kernel PLS. Further, as illustrated in Fig. 13A, in the case of Hypoxanthine, Inosine, Adenosine, and Adenine, a negative correlation (correlation coefficient of -0.6 or less) with the score of the first component is significantly recognized only for the kernel PLS-ROG.

As for the other metabolites, as illustrated in Figs. 13C and 13D, N5-Ethylglutamine (theanine) of the plasma sample and the brain sample have a significant negative correlation with the score of the first component of the kernel PLS-ROG alone. Further, as illustrated in Figs. 13B and 13C, regarding the Citrulline, only the kernel PLS and the kernel PLS-ROG of the heart sample, and the kernel PLS-ROG of the brain have a significantly negative correlation.

As described above, the data analysis apparatus 50 according to the present embodiment can generate a common score by taking into account the group order based on the kernel PLS-ROG, and thereby integrally analyze each metabolism of liver, heart, brain, and plasma samples. Further, the data analysis apparatus 50 can change the setting of the value of $\kappa$, and thereby compare the correlation in the case of the kernel PLS-ROG and the correlation in the case of the kernel PLS as described above, and perform various data analysis.

4. Conclusion

As described above, the data analysis apparatus 50 according to the present embodiment performs multivariate analysis related to a plurality of measurement items on a plurality of statistical samples based on measurement data obtained by measuring a plurality of measurement items per statistic sample. The data analysis apparatus 50 includes the storage 52 and the controller 51. The storage 52 records the data matrix X composed of measurement data obtained by measuring a plurality of measurement items per statistical sample, and the dummy matrix Y indicating group information indicating a predetermined order for a group formed by the plurality of statistical samples. The controller 51 performs predetermined calculation processing based on the data matrix X and the dummy matrix Y. The controller 51 calculates the predetermined kernel function $k(x_i, x_j)$ which uses measurement data of a pair of statistical samples in a plurality of statistical samples as the arguments $x_i$ and $x_j$. Based on the calculation result of the kernel function $k(x_i, x_j)$ and the group information, the controller 51 calculates the scores of a plurality of statistical samples according to the partial least squares method (kernel PLS-ROG) under a predetermined condition defined by the kernel matrix K and the dummy matrix Y whose matrix elements are the kernel function $k(x_i, x_j)$ per pair of statistical samples.

The data analysis apparatus 50 according to the present invention can perform integrated analysis and nonlinear analysis on various types of measurement data based on the kernel matrix K while reflecting a group order in scores based on group information (dummy matrix Y). Consequently, it is possible to perform various types of data analysis while taking into account the group order among statistical samples.

In the present embodiment, the storage 52 manages a plurality of types of measurement data $x^{(L)}_i$, $x^{(H)}_i$, $x^{(B)}_i$, and $x^{(P)}_i$ per statistical sample as the various data matrices $x_{(L)}$, $x_{(H)}$, $x_{(B)}$, and $x_{(P)}$. The various types of measurement data $x^{(L)}_i$, $x^{(H)}_i$, $x^{(B)}_i$, and $x^{(P)}_i$ are metabolome data whose measurement items are a plurality of metabolites in the biological body, for example. The controller 51 calculates the kernel matrix K from the average of the kernel functions each related to the respective type of the measurement data $x^{(L)}_i$, $x^{(H)}_i$, $x^{(B)}_i$, and $x^{(P)}_i$. Consequently, it is possible to integrally analyze a plurality of types of measurement data $x^{(L)}_i$, $x^{(H)}_i$, $x^{(B)}_i$, and $x^{(P)}_i$ which are separately managed.

In the present embodiment, the score calculated by the data analysis apparatus 50 increases or decreases according to the group order indicated by the dummy matrix Y. Therefore, by using the calculated scores, analysis of data by taking into account the group order can be facilitated. For example, in the present embodiment, the controller 51 analyzes the correlation between data of each measurement item in the measurement data and the calculated score.

In the present embodiment, the predetermined condition includes the first condition and the second condition. The first condition is a condition that the inner product via the kernel matrix K of the first vector $\alpha_x$ and itself is set to a predetermined value, the first vector $\alpha_x$ being related to an explanatory variable t of the explanatory variable t and an objective variable s in the partial least squares method (formula (13)). The second condition is a condition that the magnitude of the second vector is shifted from the predetermined value according to a predetermined penalty term based on the group information, the second vector $w_y$ being related to the objective variable s (formula (6)).

(Example)

The data analysis method (kernel PLS-ROG) according to the present invention is also useful for integrated analysis of metagenome data having an order among groups of samples, and metagenome data and metabolome data. An example of integrated analysis of metagenome data and metabolome data according to the kernel PLS-ROG will be described below.

In this example, an example will be described in which integrated analysis according to the kernel PLS-ROG is applied to metagenome data and metabolome data disclosed in Non-Patent Document 3. Non-Patent Document 3 is a research which uses metagenome data and metabolome data in breast milk of a person. Conventionally, breast milk, which is an important source of bacteria for growth of an infant, is known to influence the composition of intestinal bacteria in newborns. Non-patent Document 3 discloses that an analysis result of bacterial flora and metabolites in breast milk of a mother undergoing chemotherapy of Hodgkin's lymphoma showed the influence of chemotherapy in the profile.

According to Non-Patent Document 3, two samples of breast milk after a 0th week, a 2nd week, a 4th week, a 6th week, a 10th week, a 12th week, a 14th week, and a 16th week from a start of chemotherapy were sampled from mothers who underwent chemotherapy of the Hodgkin's lymphoma. For each sample, 16S rRNA metagenome analysis using a next generation sequencer and metabolome analysis using a gas chromatography-mass spectrometer were performed. Further, known UniFrac analysis (see, for example, Non-Patent Document 3) was performed on the data of the metagenome analysis result to obtain data constituting a similarity matrix D. The similarity matrix D is a matrix in which each element expresses the similarity between samples, and is expressed by the following formula (20) with the number of samples m.

[Math. 7]

$$D = \begin{bmatrix} 0 & d_{1,2} & \Lambda & d_{1,m} \\ d_{2,1} & 0 & \Lambda & M \\ M & M & O & d_{m-1,m} \\ d_{m,1} & \Lambda & d_{m,m-1} & 0 \end{bmatrix} \quad (20)$$

In the formula (20), $d_{i,j}$ represents a similarity (i, j = 1 to m) which is the degree of similarity between the ith sample and the jth sample. Note that di,j has a value within the range of 0 to 1, and as $d_{i,j}$ is closer to 0, the sample i and the sample j are more similar. The similarity matrix D and the metagenome analysis result data are examples of metagenome data indicating information related to the gene sequence of the bacterial flora.

Further, the metabolome data obtained by the metabolome analysis of Non-Patent Document 3 constitutes a data matrix X of 225 substances in each row and 16 samples in each column.

The statistical data of Non-Patent Document 3 as described above is open to the public. In this example, the data analysis apparatus 50 applied the kernel PLS-ROG and the kernel PLS to the statistical data of 14 samples, which is obtained by removing part of defective data from the above statistical data, to perform integrated analysis.

Based on the above similarity matrix D, the data analysis apparatus 50 generated the kernel matrix $K_g$ of metagenome data as follows: That is, each of non-diagonal elements of the kernel matrix $K_g$ was set to a reciprocal of corresponding element in the similarity matrix D. Further, each of diagonal components of the kernel matrix $K_g$ was set to 20 as a predetermined value.

In addition, the data analysis apparatus 50 generated the kernel matrix $K_m$ of the metabolome data by using the linear kernel of the data matrix X ($K_m$ = XX'). The data analysis apparatus 50 calculated the kernel matrix K obtained by integrating metagenome data and metabolome data based on the average between the kernel matrices $K_g$ and $K_m$ as in the following formula (21).

$$K = (1/2)K_g + (1/2)K_m \qquad (21)$$

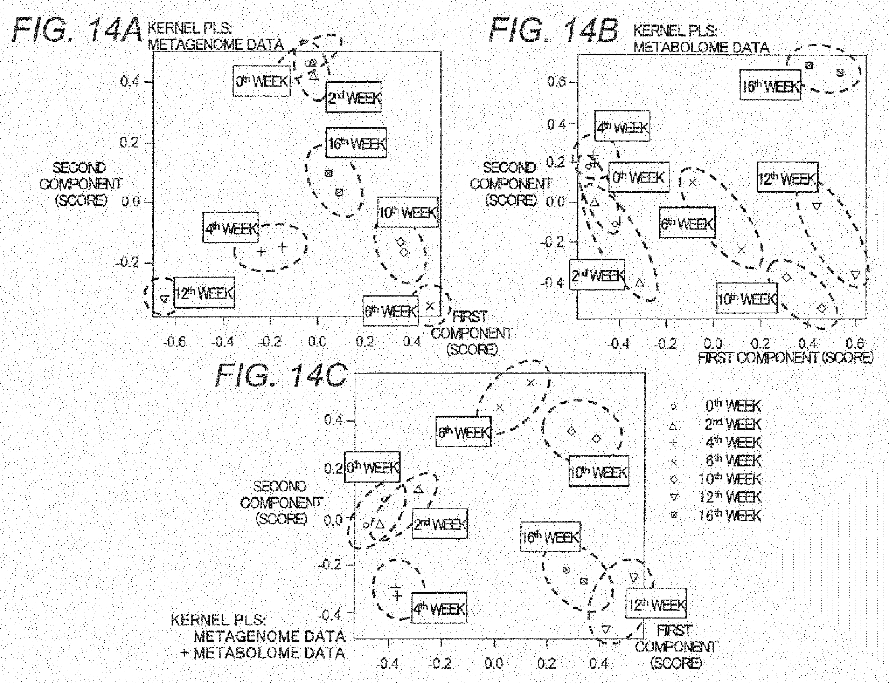

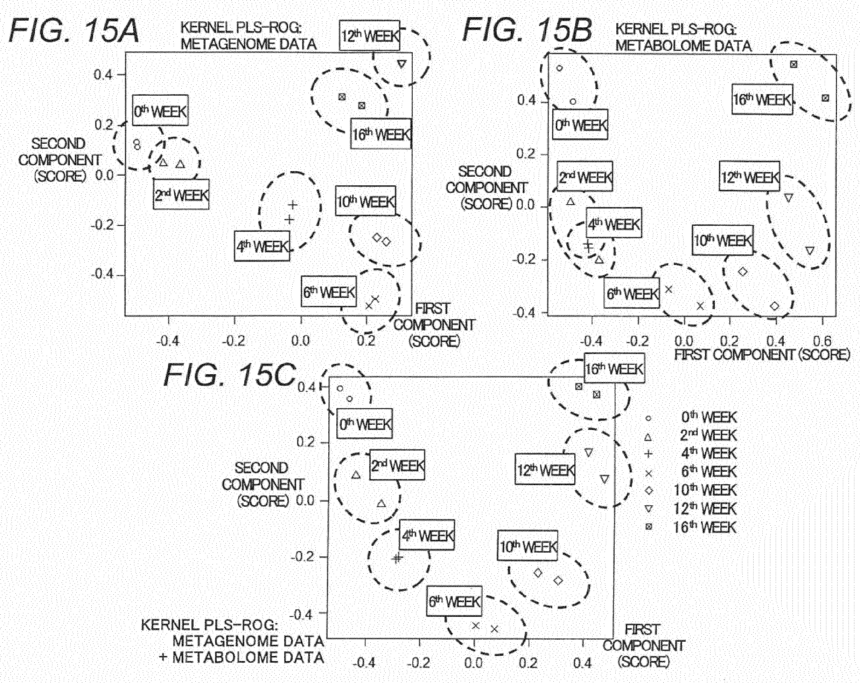

The data analysis apparatus 50 analyzed data of the kernel PLS-ROG ($\kappa$ = 0.5) and the kernel PLS ($\kappa$ = 0.5) based on the above kernel matrix K, and the dummy matrix indicating the group order of every two samples corresponding to a period from start of chemotherapy of the sample, to calculate scores. Fig. 14 and 15 illustrate analysis results.

Fig. 14A illustrates the metagenome data analysis result of the kernel PLS. Fig. 14B illustrates the metabolome data analysis result of the kernel PLS. Fig. 14C illustrates the metagenome data and metabolome data integrated analysis result of the kernel PLS. Fig. 15A illustrates the metagenome data analysis result of the kernel PLS-ROG. Fig. 15B illustrates the metabolome data analysis result of the kernel PLS-ROG. Fig. 15C illustrates the metagenome data and metabolome data integrated analysis result of the kernel PLS-ROG. In Figs. 14A to 14C and Figs. 15A to 15C, the score of each sample is plotted, horizontal axes indicate the first component of the score, and vertical axes indicate the second component of the score.

As illustrated in Figs. 14A to 14C, according to the kernel PLS, the scores per sample are not arranged in the order of the 0th to 16th weeks of a chemotherapy period in both the vertical axis and the horizontal axis. The order of the chemotherapy period does not appear in the score.

On the other hand, according to the kernel PLS-ROG, regarding the metagenome data as illustrated in Fig. 15A, the sample group of the 0th week and the sample group of the 2nd week are arranged in order in the first component (horizontal axis) of the score for example. Regarding the metagenome data as illustrated in Fig. 15B, in particular, the order of sample groups of the 6th week, the 10th week, the 12th week, and the 16th week is clear in the first component of the score. The integration result of metagenome data and metabolome data based on an average of the above shows the order among samples in each week of the 0th week, the 2nd week, the 4th week, the 6th week, the 10th week, the 12th week, and the 16th week as illustrated in Fig. 15C.

As described above, the data analysis method of the kernel PLS-ROG according to the present invention is applicable to metagenome data for analysis of bacterial flora in breast milk or bacterial flora of intestinal bacteria. The data analysis method of the kernel PLS-ROG according to the present invention can integrally analyze metagenome data and metabolome data.

(Other Embodiments)

In the first embodiment described above, an example in which a data analysis apparatus 50 is configured by an information processing device such as a PC is described. However, the present invention is not limited thereto, and the data analysis apparatus 50 may be a server device such as an ASP server. For example, the data analysis apparatus 50 may obtain, from a network interface (an example of an obtaining unit), information indicating a data matrix X and a dummy matrix Y input via a network, and execute data analysis processing. Further, the data analysis apparatus 50 may transmit information indicating a score generated by the data analysis processing via the network.

In the first embodiment, an example of application of this data analysis method to metabolomics is described. This data analysis method is not limited to metabolomics, and may be applied to various types of omics analysis and multivariate analysis of chemometrics. In this case, measurement data may be data obtained by omics analysis or chemometrics in an identical biological body.

In the first embodiment, integrated analysis of a plurality of types of metabolome data is described. This data analysis method is applicable to scenes which require various integrated analysis. This data analysis method may be used to integrate metabolome data and gene expression data, or integrate and analyze analysis data obtained from a plurality of measurement platforms.

In the first embodiment, the metabolome data illustrated in Figs. 2 to 5 is measured by using a capillary electrophoresis-time of flight mass spectrometer. An analyzing device which measures measurement data per statistical sample is not limited thereto, and may be, for example, a liquid chromatography-mass spectrometer, a gas chromatography-mass spectrometer, nuclear magnetic resonance, or the like.

In the first embodiment, the correlation of the score component selected by the user is analyzed (step S5 of Fig. 7). However, the present invention is not limited thereto, and the data analysis apparatus 50 may select score components used for analysis. For example, a controller 51 of the data analysis apparatus 50 may calculate scores, then determine the score components which reflect a group order based on the dummy matrix Y, and analyze a correlation between the determined components.

In the first embodiment, hypothesis testing is performed based on an analysis result of the data analysis processing. The data analysis apparatus 50 may perform the hypothesis testing. For example, a correlation coefficient and a threshold value of a p value may be preset to the storage 52, and the controller 51 may extract metabolites satisfying a predetermined condition (e.g., an absolute value of the correlation coefficient of "0.6" or more and the p value of "0.05" or less) to analyze the correlation between specific score components.

(Summary of Aspects)

Hereinafter, aspects of the present invention are exemplified.

1 st aspect of the present invention is a data analysis apparatus which performs multivariate analysis with a plurality of data items on a plurality of statistical samples. The data analysis apparatus comprises a storage and a controller. The storage records statistical data for managing the plurality of data items per statistical sample, and group information indicating an arrangement order of groups formed by the plurality of statistical samples. The controller performs predetermined calculation processing based on the statistical data and the group information. The controller calculates, based on the statistical data, a kernel matrix in which a matrix element represents a predetermined relationship between a statistical sample corresponding to a row number of the matrix element in the plurality of statistical samples and a statistical sample corresponding to a column number of the matrix element. The controller performs calculation processing based on a partial least squares method under a predetermined condition defined by the kernel matrix and the group information, to calculate a score for the statistical samples.

2nd aspect of the present invention is the data analysis apparatus according to 1st aspect, wherein the storage manages a plurality of types of measurement data per statistical sample in the statistical data. The controller generates a kernel matrix with respect to each of the plurality of types of measurement data, and calculates an integrated kernel matrix based on an average of kernel matrices for the plurality of types of measurement data.

3rd aspect of the present invention is the data analysis apparatus according to 1st or 2nd aspect, wherein the predetermined relationship is defined by a kernel function based on data with respect to the statistical sample corresponding to the row number in the statistical data, and data with respect to the statistical sample corresponding to the column number.

4th aspect of the present invention is the data analysis apparatus according to any one of 1st to 3rd aspects, wherein the score increases or decreases according to the order of the groups indicated by the group information.

5th aspect of the present invention is the data analysis apparatus according to any one of 1st to 4th aspects, wherein the controller analyzes a correlation between data per data item in the statistical data and the calculated score.

6th aspect of the present invention is the data analysis apparatus according to any one of 1 st to 5th aspects, wherein the predetermined condition includes a first condition and a second condition. The first condition is for setting an inner product via the kernel matrix between a first vector and itself to a predetermined value, the first vector being related to an explanatory variable of the explanatory variable and an objective variable in the partial least squares method. The second condition is for shifting a magnitude of a second vector from a predetermined value by a predetermined penalty term based on the group information, the second vector being related to the objective variable.

7th aspect of the present invention is the data analysis apparatus according to any one of 1st to 6th aspects, wherein the statistical data includes metabolome data in which the data items are a plurality of metabolites in biological bodies.

8th aspect of the present invention is the data analysis apparatus according to any one of 1st to 7th aspects, wherein the statistical data includes metagenome data indicating information related to a gene sequence of a bacterial flora.

9th aspect of the present invention is the data analysis apparatus according to any one of 1st to 8th aspects, wherein the statistical data includes data obtained by omics analysis or chemometrics in an identical biological body.

10th aspect of the present invention is a data analysis method for causing a computer to perform multivariate analysis with a plurality of data items on a plurality of statistical samples. A storage of the computer records statistical data for managing the plurality of data items per statistical sample, and group information indicating an arrangement order of groups formed by the plurality of statistical samples. The data analysis method comprises, by the computer, calculating, based on the statistical data, a kernel matrix in which a matrix element represents a predetermined relationship between a statistical sample corresponding to a row number of the matrix element in the plurality of statistical samples and a statistical sample corresponding to a column number of the matrix element. The data analysis method comprises, by the computer, performing calculation processing based on a partial least squares method under a predetermined condition defined by the kernel matrix and the group information, to calculate a score for the statistical samples.

11th aspect of the present invention is a program for causing a computer to execute the data analysis method according to 10th aspect.

**Claims**

1. A data analysis apparatus which performs multivariate analysis with a plurality of data items on a plurality of statistical samples, the data analysis apparatus comprising:

   a storage which records statistical data for managing the plurality of data items per statistical sample, and group information indicating an arrangement order of groups formed by the plurality of statistical samples; and
   a controller which performs predetermined calculation processing based on the statistical data and the group information,
   wherein the controller
   calculates, based on the statistical data, a kernel matrix in which a matrix element represents a predetermined relationship between a statistical sample corresponding to a row number of the matrix element in the plurality of statistical samples and a statistical sample corresponding to a column number of the matrix element, and
   performs calculation processing based on a partial least squares method under a predetermined condition defined by the kernel matrix and the group information, to calculate a score for the statistical samples.

2. The data analysis apparatus according to claim 1, wherein
   the storage manages a plurality of types of measurement data per statistical sample in the statistical data, and
   the controller
   generates a kernel matrix with respect to each of the plurality of types of measurement data, and
   calculates an integrated kernel matrix based on an average of kernel matrices for the plurality of types of measurement data.

3. The data analysis apparatus according to claim 1 or 2, wherein the predetermined relationship is defined by a kernel function based on data with respect to the statistical sample corresponding to the row number in the statistical data, and data with respect to the statistical sample corresponding to the column number.

4. The data analysis apparatus according to any one of claims 1 to 3, wherein the score increases or decreases according to the order of the groups indicated by the group information.

5. The data analysis apparatus according to any one of claims 1 to 4, wherein the controller analyzes a correlation between data per data item in the statistical data and the calculated score.

6. The data analysis apparatus according to any one of claims 1 to 5, wherein
   the predetermined condition includes
   a first condition for setting an inner product via the kernel matrix between a first vector and itself to a predetermined value, the first vector being related to an explanatory variable of the explanatory variable and an objective variable in the partial least squares method, and
   a second condition for shifting a magnitude of a second vector from a predetermined value by a predetermined penalty term based on the group information, the second vector being related to the objective variable.

7. The data analysis apparatus according to any one of claims 1 to 6, wherein the statistical data includes metabolome data in which the data items are a plurality of metabolites in biological bodies.

8. The data analysis apparatus according to any one of claims 1 to 7, wherein the statistical data includes metagenome data indicating information related to a gene sequence of a bacterial flora.

9. The data analysis apparatus according to any one of claims 1 to 8, wherein the statistical data includes data obtained by omics analysis or chemometrics in an identical biological body.

10. A data analysis method for causing a computer to perform multivariate analysis with a plurality of data items on a plurality of statistical samples, wherein
a storage of the computer records statistical data for managing the plurality of data items per statistical sample, and group information indicating an arrangement order of groups formed by the plurality of statistical samples, and the data analysis method comprises, by the computer:

calculating, based on the statistical data, a kernel matrix in which a matrix element represents a predetermined relationship between a statistical sample corresponding to a row number of the matrix element in the plurality of statistical samples and a statistical sample corresponding to a column number of the matrix element; and performing calculation processing based on a partial least squares method under a predetermined condition defined by the kernel matrix and the group information, to calculate a score for the statistical samples.

11. A program for causing a computer to execute the data analysis method according to claim 10.

## FIG. 1A

BRAIN SAMPLE 1
METABOLOME DATA

β-Ala,
Xanthosine,
Xanthine,
Val,
Uridine, Urate,
Uracil, ...

INDIVIDUAL 2

INDIVIDUAL n

INDIVIDUAL 1

HEART SAMPLE 1
METABOLOME DATA

1,4-Butanediamine,
10-Hydroxydecanoic acid,
1-Metylhistamine,
2,3-Diphosphoglyceric acid,
2-Aminobutyrate,
2-Deoxyglucose 6-phosphate,
2-Hydroxypentanoic acid, ...

LIVER SAMPLE 1
METABOLOME DATA

2-Hydroxypentanoic acid,
2-Methylserine,
2-Oxoisovaleric acid,
3-Hydroxy-3-methylglutaric acid,
3-Hydroxybutyric acid,
3-Hydroxypropionic acid,
3-Methylhistidine,
3-Phenylpropionic acid, ...

DATA
INPUT

## FIG. 1B

50

DATA ANALYSIS APPARATUS

SECOND
COMPONENT          SCORE          ◆ SAMPLE

5

4

3

2

1

0

0          2          4

FIRST
COMPONENT

# FIG. 2

$X_{(L)}'$  $X_1^{(L)}$

| METABOLITES OF LIVER | SAMPLE 1 | SAMPLE 2 | SAMPLE 3 | ... | SAMPLE 8 | SAMPLE 9 |
|---|---|---|---|---|---|---|
| 1,4-Butanediamine | 1.29E-03 | 1.04E-03 | 7.40E-04 | ... | 9.25E-04 | 7.50E-04 |
| 1-Methylnicotinamide | 7.74E-04 | 8.25E-04 | 8.41E-04 | ... | 8.98E-04 | 6.62E-04 |
| 1-Metylhistamine | 2.34E-04 | 2.79E-04 | 1.90E-04 | ... | 2.14E-04 | 4.43E-04 |
| 2,3-Diaminopropionate | 7.61E-04 | 1.13E-03 | 7.46E-04 | ... | 1.16E-03 | 9.13E-04 |
| 2,3-Diphosphoglyceric acid | 5.43E-03 | 1.31E-02 | 4.71E-03 | ... | 6.63E-03 | 2.29E-02 |
| 2-Aminobutyrate | 1.51E-02 | 2.11E-02 | 1.73E-02 | ... | 2.77E-02 | 2.00E-02 |
| 2'-Deoxyguanosine | 3.67E-04 | 5.28E-04 | 5.03E-04 | ... | 4.10E-04 | 3.41E-04 |
| 2-Hydroxyisobutyric acid, 2-Hydroxybutyric acid, 3-Hydroxybutyric acid | 1.05E-02 | 1.70E-02 | 1.46E-02 | ... | 1.55E-02 | 1.99E-02 |
| 2-Hydroxypentanoic acid | 9.09E-03 | 1.31E-02 | 1.20E-02 | ... | 1.38E-02 | 1.20E-02 |
| 2-Methylserine | 5.64E-04 | 1.61E-03 | 1.60E-03 | ... | 7.53E-04 | 1.25E-03 |
| 2-Oxoisovaleric acid | 7.03E-03 | 9.64E-03 | 8.62E-03 | ... | 6.74E-03 | 7.05E-03 |
| 3-Hydroxypropionic acid | 6.17E-02 | 1.05E-01 | 8.90E-02 | ... | 5.59E-02 | 8.92E-02 |
| 5-Methylthioadenosine | 4.81E-04 | 1.37E-03 | 8.72E-04 | ... | 7.57E-04 | 1.29E-03 |
| 5-Oxoproline | 1.32E-02 | 1.41E-02 | 1.29E-02 | ... | 1.62E-02 | 1.35E-02 |
| 6-Phosphogluconic acid | 1.14E-02 | 1.07E-02 | 1.88E-02 | ... | 6.37E-03 | 1.85E-02 |
| N,N-Dimethylglycine | 1.06E-02 | 1.37E-02 | 1.13E-02 | ... | 4.45E-02 | 7.37E-02 |
| a-Aminoadipate | 4.35E-02 | 4.61E-02 | 2.97E-02 | ... | 3.94E-02 | 3.62E-02 |
| Adenine | 1.71E-02 | 2.18E-02 | 1.92E-02 | ... | 1.42E-02 | 1.21E-02 |
| Adenosine | 1.73E-02 | 1.87E-02 | 2.29E-02 | ... | 1.29E-02 | 9.95E-03 |
| Adenylosuccinic acid | 2.49E-02 | 8.01E-02 | 5.65E-02 | ... | 3.45E-02 | 2.05E-02 |
| Adipic acid | 2.30E-03 | 2.39E-03 | 2.19E-03 | ... | 4.23E-03 | 2.94E-03 |
| ADP | 8.41E-02 | 1.28E-01 | 9.96E-02 | ... | 6.70E-02 | 1.15E-01 |
| ADPribose | 1.09E-02 | 1.75E-02 | 5.84E-03 | ... | 9.22E-03 | 4.61E-02 |
| Ala | 6.81E-01 | 1.38E+00 | 6.51E-01 | ... | 6.25E-01 | 1.04E+00 |
| Ala-Ala | 3.47E-03 | 2.38E-03 | 1.80E-03 | ... | 2.19E-03 | 1.40E-03 |
| Arg | 1.10E-02 | 3.06E-02 | 1.36E-02 | ... | 6.72E-03 | 1.25E-02 |
| Argininosuccinic acid | 3.82E-03 | 1.50E-02 | 7.24E-03 | ... | 3.51E-03 | 6.78E-03 |
| Asn | 2.04E-01 | 2.36E-01 | 2.06E-01 | ... | 1.39E-01 | 1.85E-01 |
| Asp | 7.12E-01 | 1.23E+00 | 5.87E-01 | ... | 6.58E-01 | 5.87E-01 |
| ATP | 6.92E-03 | 1.18E-02 | 1.08E-02 | ... | 4.98E-03 | 1.33E-02 |
| Benzoic acid | 3.46E-03 | 3.98E-03 | 3.28E-03 | ... | 3.54E-03 | 3.84E-03 |
| Betaine | 2.19E-01 | 3.44E-01 | 2.15E-01 | ... | 9.97E-01 | 7.04E-01 |
| Betaine aldehyde_H2O | 7.30E-03 | 1.56E-02 | 3.66E-03 | ... | 5.97E-03 | 3.86E-03 |
| Butanoic acid | 1.66E-03 | 3.40E-03 | 2.78E-03 | | 2.98E-03 | 7.98E-03 |
| Hypoxanthine | 7.09E-02 | 7.39E-02 | 7.68E-02 | 4.30E-02 | 4.44E-02 | 3.83E-02 |
| Inosine | 3.74E-01 | 3.95E-01 | 4.17E-01 | 2.20E-01 | 2.26E-01 | 1.97E-01 |
| Urate | 1.02E-03 | 2.29E-03 | 3.75E-03 | 7.76E-04 | 2.64E-02 | 2.07E-02 |
| CDP-choline | 1.27E-03 | | | | | |

# FIG. 3

$X_{(H)}'$     $X_1^{(H)}$

| METABOLITES OF HEART | SAMPLE 1 | SAMPLE 2 | SAMPLE 3 | · · · | SAMPLE 8 | SAMPLE 9 |
|---|---|---|---|---|---|---|
| 1,4-Butanediamine | 1.47E-02 | 9.74E-03 | 8.79E-03 | · · · | 8.09E-03 | 1.06E-02 |
| 10-Hydroxydecanoic acid | 1.29E-03 | 3.86E-03 | 1.21E-03 | · · · | 1.45E-03 | 1.07E-03 |
| 1-Metylhistamine | 3.83E-04 | 3.37E-04 | 1.76E-04 | · · · | 2.32E-04 | 3.81E-04 |
| 2,3-Diphosphoglyceric acid | 2.19E-03 | 5.59E-03 | 2.57E-03 | · · · | 3.18E-03 | 5.70E-03 |
| 2-Aminobutyrate | 3.71E-03 | 6.31E-03 | 5.26E-03 | · · · | 6.34E-03 | 6.53E-03 |
| 2-Deoxyglucose 6-phosphate | 1.00E-03 | 1.30E-03 | 8.35E-04 | · · · | 8.31E-04 | 9.58E-04 |
| 2-Hydroxyisobutyric acid, 2-Hydroxybutyric acid, 3-Hydroxybutyric acid | 7.71E-03 | 2.26E-02 | 5.62E-03 | · · · | 1.16E-02 | 5.69E-03 |
| 2-Hydroxypentanoic acid | 5.86E-03 | 1.65E-02 | 7.72E-03 | · · · | 9.08E-03 | 6.01E-03 |
| 3-Guanidinopropionate | 3.81E-03 | 1.77E-03 | 2.20E-03 | · · · | 2.12E-03 | 4.94E-03 |
| 3-Hydroxy-3-methylglutaric acid | 4.62E-04 | 4.68E-04 | 4.31E-04 | · · · | 6.08E-04 | 4.37E-04 |
| 3-Hydroxybutyric acid | 1.81E-01 | 3.66E-01 | 2.27E-01 | · · · | 2.76E-01 | 2.60E-01 |
| 3-Methylhistidine | 1.15E-01 | 8.72E-02 | 9.40E-02 | · · · | 6.56E-02 | 5.21E-02 |
| 3-Phenylpropionic acid | 4.73E-03 | 4.94E-03 | 3.92E-03 | · · · | 2.94E-03 | 5.35E-03 |
| 3-Phosphoglyceric acid | 2.81E-03 | 3.56E-03 | 3.80E-03 | · · · | 2.48E-03 | 2.31E-03 |
| 5-Oxoproline | 2.88E-02 | 2.89E-02 | 2.81E-02 | · · · | 3.08E-02 | 4.49E-02 |
| 6-Hydroxyhexanoic acid | 5.49E-03 | 2.24E-02 | 6.59E-03 | · · · | 6.21E-03 | 5.66E-03 |
| N,N-Dimethylglycine | 1.45E-03 | 1.64E-03 | 2.09E-03 | · · · | 4.81E-03 | 5.55E-03 |
| N5-Ethylglutamine | 2.36E-03 | 3.58E-03 | 4.01E-03 | · · · | 2.64E-03 | 3.96E-03 |
| a-Aminoadipate | 4.37E-02 | 5.76E-02 | 4.82E-02 | · · · | 4.87E-02 | 6.43E-02 |
| Acetyl CoA | 2.68E-03 | 3.62E-03 | 6.18E-03 | · · · | 5.44E-03 | 1.14E-02 |
| Acetylcholine | 1.59E-03 | 9.36E-04 | 2.48E-03 | · · · | 5.74E-04 | 4.96E-04 |
| Adenine | 4.47E-02 | 4.72E-02 | 4.90E-02 | · · · | 5.53E-02 | 4.76E-02 |
| Adenosine | 3.72E-02 | 5.68E-02 | 8.48E-01 | · · · | 1.45E-01 | 6.35E-01 |
| Adenylosuccinic acid | 2.38E-02 | 3.67E-02 | 3.99E-02 | · · · | 7.28E-02 | 5.99E-02 |
| Adipic acid | 8.59E-04 | 8.01E-04 | 7.25E-04 | · · · | 8.02E-04 | 5.26E-04 |
| ADP | 3.42E-01 | 2.39E-01 | 3.08E-01 | · · · | 2.83E-01 | 3.27E-01 |
| Ala | 3.75E+00 | 4.50E+00 | 4.13E+00 | · · · | 4.18E+00 | 4.24E+00 |
| Ala-Ala | 2.11E-03 | 2.16E-03 | 1.92E-03 | · · · | 2.13E-03 | 1.78E-03 |
| AMP | 1.44E+00 | 1.40E+00 | 1.57E+00 | · · · | 1.49E+00 | 1.79E+00 |
| Anserine, Homocarnosine | 2.22E-01 | 1.40E-01 | 2.26E-01 | · · · | 2.38E-01 | 2.52E-01 |
| Anthranilate | 1.21E-03 | 1.51E-03 | 1.65E-03 | · · · | 1.58E-03 | 2.03E-03 |
| Arg | 4.50E-01 | 5.65E-01 | 5.97E-01 | · · · | 5.37E-01 | 5.80E-01 |
| Argininosuccinic acid | 1.54E-03 | 2.65E-03 | 2.39E-03 | · · · | 2.96E-03 | 4.68E-03 |
| Betaine | 4.30E-01 | 8.26E-01 | 1.73E+00 | · · · | 3.30E+00 | 1.76E+00 |
| Citrulline | 1.10E-01 | 1.66E-01 | 9.37E-02 | · · · | 3.88E-02 | 4.47E-02 |
| CMP | 2.91E-03 | 3.22E-03 | 3.08E-03 | · · · | 4.05E-03 | 3.15E-03 |
| CoA | 1.68E-02 | 2.18E-02 | | | | |

# FIG. 4

$X_{(B)}'$     $x_1^{(B)}$

| METABOLITES OF BRAIN | SAMPLE 1 | SAMPLE 2 | SAMPLE 3 | · · · | SAMPLE 8 | SAMPLE 9 |
|---|---|---|---|---|---|---|
| β-Ala | 6.77E-02 | 7.68E-02 | 7.73E-02 | · · · | 8.57E-02 | 7.07E-02 |
| Xanthosine | 2.70E-03 | 1.29E-03 | 1.61E-03 | · · · | 1.62E-03 | 1.06E-03 |
| Xanthine | 5.53E-03 | 5.77E-03 | 5.65E-03 | · · · | 7.02E-03 | 5.00E-03 |
| Val | 3.92E-01 | 3.76E-01 | 3.43E-01 | · · · | 3.72E-01 | 3.27E-01 |
| Uridine | 1.13E-01 | 7.50E-02 | 9.03E-02 | · · · | 1.08E-01 | 6.83E-02 |
| N8-Acetylspermidine | 3.15E-04 | 2.25E-04 | 2.29E-04 | · · · | 3.83E-04 | 2.72E-04 |
| N6-Acetyllysine | 1.45E-03 | 1.40E-03 | 1.50E-03 | · · · | 1.40E-03 | 1.15E-03 |
| N6,N6,N6-Trimethyllysine | 5.65E-03 | 5.52E-03 | 5.52E-03 | · · · | 7.27E-03 | 6.28E-03 |
| N5-Ethylglutamine | 7.44E-03 | 6.35E-03 | 7.17E-03 | · · · | 3.36E-03 | 3.19E-03 |
| Trp | 3.78E-02 | 3.30E-02 | 3.19E-02 | · · · | 3.34E-02 | 3.07E-02 |
| Trimethylamine N-oxide | 8.04E-04 | 9.06E-04 | 4.59E-03 | · · · | 1.43E-03 | 2.21E-03 |
| Threonic acid | 7.83E-02 | 7.59E-02 | 8.57E-02 | · · · | 1.08E-01 | 6.23E-02 |
| Thr | 5.18E-01 | 5.21E-01 | 3.87E-01 | · · · | 4.03E-01 | 4.83E-01 |
| Taurine | 3.69E-01 | 4.61E-01 | 3.51E-01 | · · · | 3.97E-01 | 5.37E-01 |
| Succinic acid | 2.68E-01 | 2.86E-01 | 1.23E-01 | · · · | 2.38E-01 | 2.54E-01 |
| Spermine | 1.96E-03 | 1.73E-03 | 7.42E-04 | · · · | 1.62E-03 | 7.47E-04 |
| Spermidine | 1.78E-02 | 1.88E-02 | 1.26E-02 | · · · | 1.72E-02 | 1.81E-02 |
| S-Lactoylglutathione | 8.18E-04 | 1.25E-03 | 1.02E-03 | · · · | 1.01E-03 | 1.26E-03 |
| Serotonin | 1.34E-03 | 1.79E-03 | 1.44E-03 | · · · | 2.55E-03 | 1.41E-03 |
| Ser | 1.23E+00 | 1.41E+00 | 1.39E+00 | · · · | 1.48E+00 | 1.11E+00 |
| Sedoheptulose 7-phosphate | 6.72E-03 | 5.68E-03 | 4.36E-03 | · · · | 5.67E-03 | 6.39E-03 |
| Sarcosine | 9.78E-04 | 1.19E-03 | 1.78E-03 | · · · | 1.60E-03 | 1.39E-03 |
| SAM | 1.53E-02 | 1.66E-02 | 1.49E-02 | · · · | 2.01E-02 | 1.99E-02 |
| SAH | 5.30E-03 | 5.23E-03 | 4.55E-03 | · · · | 5.29E-03 | 5.77E-03 |
| CMP | 5.85E-03 | 8.74E-03 | 7.15E-03 | · · · | 6.82E-03 | 4.10E-03 |
| Citrulline | 1.58E-01 | 1.38E-01 | 1.34E-01 | · · · | 8.80E-02 | 6.39E-02 |
| Citrate | 2.64E-01 | 1.49E-01 | 4.11E-01 | · · · | 2.79E-01 | 1.30E-01 |
| Pyridoxamine 5'-phosphate | 9.82E-03 | 1.11E-02 | 9.21E-03 | · · · | 1.17E-02 | 8.32E-03 |
| Prostaglandin F2a | 3.87E-03 | 2.41E-03 | 4.48E-03 | · · · | 4.88E-03 | 2.17E-03 |
| Propionic acid | 3.16E-03 | 4.05E-03 | 3.63E-03 | · · · | 3.88E-03 | 4.21E-03 |
| Pro | 3.11E-01 | 2.73E-01 | 2.46E-01 | · · · | 3.34E-01 | 2.82E-01 |
| Pipecolate | 1.02E-03 | 2.09E-03 | 3.01E-03 | · · · | 2.42E-03 | 5.92E-03 |
| Phosphorylcholine | 5.32E-01 | 5.62E-01 | 5.99E-01 | · · · | 6.24E-01 | 5.37E-01 |
| Phe | 2.26E-01 | 1.99E-01 | 2.04E-01 | · · · | 2.03E-01 | 1.67E-01 |
| Pelargonic acid | 5.71E-03 | | 5.73E-03 | · · · | 5.59E-03 | 7.82E-03 |
| Pantothenic acid | 2.91E-02 | 3.40E-02 | 2.54E-02 | · · · | 3.76E-02 | 2.66E-02 |
| Ornithine | 3.35E-02 | 3.33E-02 | 2.32E-02 | · · · | 3.29E-02 | 3.94E-02 |
| O-Acetylcarnitine | 3.36E-02 | 4.00E-02 | 6.99E-02 | · · · | 6.23E-02 | 9.19E-02 |
| Nicotinamide, | | | | | | |

# FIG. 5

$X_{(P)}'$    $X_1^{(P)}$

| METABOLITES OF PLASMA | SAMPLE 1 | SAMPLE 2 | SAMPLE 3 | ... | SAMPLE 8 | SAMPLE 9 |
|---|---|---|---|---|---|---|
| 2,3-Diphosphoglyceric acid | 1.38E-02 | 5.99E-03 | 5.97E-03 | ... | 8.62E-03 | 9.19E-03 |
| 2-Aminobutyrate | 6.35E-02 | 7.13E-02 | 5.20E-02 | ... | 6.82E-02 | 7.05E-02 |
| 2-Hydroxyisobutyric acid, 2-Hydroxybutyric acid, 3-Hydroxybutyric acid | 1.68E-01 | 1.69E-01 | 1.30E-01 | ... | 1.58E-01 | 1.34E-01 |
| 2-Hydroxypentanoic acid | 8.26E-02 | 7.54E-02 | 9.71E-02 | ... | 7.62E-02 | 1.14E-01 |
| 2-Oxoglutaric acid | 9.80E-02 | 5.86E-02 | 5.77E-02 | ... | 6.68E-02 | 9.12E-02 |
| 2-Oxoisopentanoic acid | 7.09E-02 | 6.82E-02 | 5.83E-02 | ... | 6.55E-02 | 7.70E-02 |
| 3-Hydroxybutyric acid | 1.30E+00 | 1.04E+00 | 5.66E-01 | ... | 1.26E+00 | 6.85E-01 |
| 3-Methylhistidine | 2.91E-01 | 4.68E-01 | 3.83E-01 | ... | 3.06E-01 | 2.63E-01 |
| 3-Phenylpropionic acid | 2.87E-02 | 4.42E-02 | 2.40E-02 | ... | 3.24E-02 | 4.72E-02 |
| 3-Phosphoglyceric acid | 8.11E-03 | 1.05E-02 | 6.90E-03 | ... | 8.31E-03 | 6.32E-03 |
| 4-Acetylbutyric acid | 1.99E-02 | 2.07E-02 | 1.73E-02 | ... | 1.76E-02 | 1.97E-02 |
| 4-Methyl-2-oxopentanoic acid | 3.24E-01 | 3.54E-01 | 2.55E-01 | ... | 3.33E-01 | 2.87E-01 |
| 4-Oxopentanoic acid | 1.59E-02 | 2.07E-02 | 1.49E-02 | ... | 1.24E-02 | 1.45E-02 |
| 4-Pyridoxic acid | 3.48E-03 | 3.49E-03 | 3.58E-03 | ... | 5.02E-03 | 2.92E-03 |
| 5-Oxoproline | 7.56E-02 | 8.16E-02 | 8.19E-02 | ... | 8.13E-02 | 8.62E-02 |
| 6-Hydroxyhexanoic acid | 9.06E-03 | 1.08E-02 | 9.22E-03 | ... | 1.08E-02 | 1.22E-02 |
| N,N-Dimethylglycine | 6.26E-02 | 1.54E-02 | 1.94E-02 | ... | 6.05E-02 | 7.20E-02 |
| N5-Ethylglutamine | 2.18E-02 | 3.72E-02 | 3.52E-02 | ... | 1.51E-02 | 1.93E-02 |
| N6,N6,N6-Trimethyllysine | 2.20E-02 | 3.18E-02 | 2.44E-02 | ... | 2.22E-02 | 3.20E-02 |
| Anserine, Homocarnosine | 8.74E-03 | 7.70E-03 | 7.36E-03 | ... | 8.49E-03 | 7.23E-03 |
| Anthranilate | 1.72E-02 | 2.58E-02 | 1.83E-02 | ... | 1.28E-02 | 1.93E-02 |
| Arg | 1.96E+00 | 2.51E+00 | 1.79E+00 | ... | 1.93E+00 | 2.16E+00 |
| Argininosuccinic acid | 3.85E-03 | 4.47E-03 | 4.85E-03 | ... | 2.40E-03 | 3.67E-03 |
| Asn | 3.09E-01 | 4.45E-01 | 2.69E-01 | ... | 3.43E-01 | 4.10E-01 |
| Asp | 5.97E-02 | 5.98E-02 | 6.64E-02 | ... | 6.75E-02 | 3.83E-02 |
| Azelaic acid | 5.66E-03 | 5.34E-03 | 5.35E-03 | ... | 4.47E-03 | 6.87E-03 |
| Benzoic acid | 2.51E-02 | 2.57E-02 | 2.50E-02 | ... | 2.55E-02 | 2.82E-02 |
| Betaine | 6.31E+00 | 2.79E+00 | 2.39E+00 | ... | 5.18E+00 | 4.67E+00 |
| Butanoic acid | 4.77E-02 | 1.12E-01 | 4.27E-02 | ... | 3.32E-02 | 1.06E-01 |
| Carnitine | 5.65E-01 | 5.69E-01 | 5.72E-01 | ... | 5.13E-01 | 5.81E-01 |
| Carnosine | 3.17E-02 | 2.18E-02 | 1.43E-02 | ... | 2.33E-02 | 4.00E-02 |
| Choline | 4.32E-01 | 4.15E-01 | 3.84E-01 | ... | 4.33E-01 | 3.85E-01 |
| cis-Aconitic acid | 1.01E-01 | 7.20E-02 | 6.79E-02 | ... | 8.72E-02 | 1.07E-01 |
| Citramalic acid | 9.10E-03 | 5.54E-03 | 4.79E-03 | ... | 4.82E-03 | 9.53E-03 |
| Citrate | 1.75E+00 | 1.75E+00 | 1.65E+00 | ... | 1.66E+00 | 2.20E+00 |
| Citrulline | 7.52E-01 | 1.20E+00 | 8.43E-01 | ... | 5.96E-01 | 7.22E-01 |
| Creatine | 6.23E-01 | 4.90E-01 | 4.59E-01 | ... | 2.67E-01 | 8.01E-01 |
| Creatinine | 9.03E-01 | 1.05E+00 | 9.61E-01 | ... | 8.97E-01 | 1.01E+00 |
| Cys | | | | | | |

FIG. 6

DATA ANALYSIS APPARATUS 50

53 OPERATION UNIT

54 DISPLAY

55 DEVICE INTERFACE

51 CONTROLLER

56 NETWORK INTERFACE

52 STORAGE

PROGRAM    DATA

## FIG. 7

```
        ┌─────────────────────┐
        │   DATA ANALYSIS     │
        │    PROCESSING       │
        └─────────────────────┘
                  │
  ┌───────────────────────────────────┐
S1│  OBTAIN DATA MATRICES             │
  │  INDICATING METABOLOME           │
  │  DATA FOR EVERY ORGAN            │
  └───────────────────────────────────┘
                  │
  ┌───────────────────────────────────┐
S2│  OBTAIN A DUMMY MATRIX            │
  │  INDICATING GROUP                │
  │  INFORMATION                     │
  └───────────────────────────────────┘
                  │
  ┌───────────────────────────────────┐
S3│  │  KERNEL PLS-ROG        │       │
  │  │  CALCULATION          │       │
  │  │  PROCESSING           │       │
  └───────────────────────────────────┘
                  │
  ┌───────────────────────────────────┐
S4│  DISPLAY SCORES FOR EACH         │
  │  SAMPLE                          │
  └───────────────────────────────────┘
                  │
S5            ◇ IS A                No
          COMPONENT  ───────────────┐
           SELECTED ?               │
              │                     │
             Yes                    │
  ┌───────────────────────────────────┐
S6│  ANALYZE A CORRELATION           │
  │  WITH METABOLITES FOR            │
  │  EACH ORGAN                      │
  └───────────────────────────────────┘
                  │←───────────────────┘
        ┌─────────────────────┐
        │        END          │
        └─────────────────────┘
```

## FIG. 8A

METABOLITES

$$\mathbf{X}_{(L)} = \begin{bmatrix} \mathbf{x}_1^{(L)\prime} \\ \mathbf{x}_2^{(L)\prime} \\ \vdots \\ \mathbf{x}_9^{(L)\prime} \end{bmatrix}$$ SAMPLE NUMBER

## FIG. 8B

GROUPS

$$\mathbf{Y} = \begin{bmatrix} 1 & 0 & 0 \\ 1 & 0 & 0 \\ 1 & 0 & 0 \\ 0 & 1 & 0 \\ 0 & 1 & 0 \\ 0 & 1 & 0 \\ 0 & 0 & 1 \\ 0 & 0 & 1 \\ 0 & 0 & 1 \end{bmatrix}$$ SAMPLE NUMBER

## FIG. 8C

SAMPLE NUMBER

$$\mathbf{K}_{(L)} = \begin{bmatrix} \mathbf{x}_1^{(L)\prime}\mathbf{x}_1^{(L)} & \mathbf{x}_1^{(L)\prime}\mathbf{x}_2^{(L)} & \cdots & \mathbf{x}_1^{(L)\prime}\mathbf{x}_9^{(L)} \\ \mathbf{x}_2^{(L)\prime}\mathbf{x}_1^{(L)} & \mathbf{x}_2^{(L)\prime}\mathbf{x}_2^{(L)} & & \vdots \\ \vdots & & \ddots & \\ \mathbf{x}_9^{(L)\prime}\mathbf{x}_1^{(L)} & \cdots & & \mathbf{x}_9^{(L)\prime}\mathbf{x}_9^{(L)} \end{bmatrix}$$ SAMPLE NUMBER

## FIG. 8D

$$\mathbf{K} = \frac{1}{4}\mathbf{K}_{(L)} + \frac{1}{4}\mathbf{K}_{(H)} + \frac{1}{4}\mathbf{K}_{(B)} + \frac{1}{4}\mathbf{K}_{(P)}$$

## FIG. 8E

SAMPLE NUMBER

$$\mathbf{P} = \begin{bmatrix} 1/3 & 1/3 & 1/3 & 0 & 0 & 0 & 0 & 0 & 0 \\ 0 & 0 & 0 & 1/3 & 1/3 & 1/3 & 0 & 0 & 0 \\ 0 & 0 & 0 & 0 & 0 & 0 & 1/3 & 1/3 & 1/3 \end{bmatrix}$$ GROUPS

## FIG. 8F

GROUPS

$$\mathbf{D} = \begin{bmatrix} -1 & 1 & 0 \\ 0 & -1 & 1 \end{bmatrix}$$ GROUPS − 1

*FIG. 9*

```
        ┌─────────────────────┐
        │  KERNEL PLS-ROG      │
        │  CALCULATION         │
        │  PROCESSING          │
        └─────────────────────┘
                  │
     ┌──────────────────────────────┐
S10  │  PERFORM SCALING OF DATA     │
     │     OF METABOLITES           │
     └──────────────────────────────┘
                  │
     ┌──────────────────────────────┐
S11  │  SELECT A TYPE               │
     │  SUCH AS ORGANS              │
     └──────────────────────────────┘
                  │
     ┌──────────────────────────────┐
S12  │  CALCULATE                   │
     │  KERNEL FUNCTION             │
     │  BY ONE MATRIX ELEMENT       │
     └──────────────────────────────┘
                  │
            ◇ ARE ALL MATRIX
S13         ELEMENTS          ──── No
            CALCULATED ? ◇
                  │
                 Yes
                  │
            ◇ IS THE
S14         CALCULATION       ──── No
            DONE IN ALL
            TYPES ? ◇
                  │
                 Yes
                  │
     ┌──────────────────────────────┐
S15  │  GENERATE A KERNEL MATRIX    │
     │  BY AVERAGE OVER TYPES       │
     └──────────────────────────────┘
                  │
     ┌──────────────────────────────┐
     │  SUBSTITUTE THE KERNEL       │
S16  │  MATRIX AND A DUMMY MATRIX   │
     │  IN ARITHMETIC EXPRESSION OF │
     │  KERNEL PLS-ROG              │
     └──────────────────────────────┘
                  │
     ┌──────────────────────────────┐
S17  │  CALCULATE EIGEN VALUES AND  │
     │  EIGEN VECTORS               │
     └──────────────────────────────┘
                  │
     ┌──────────────────────────────┐
S18  │  CALCULATE SCORES            │
     │  BASED ON THE EIGEN VECTORS  │
     └──────────────────────────────┘
                  │
           ┌──────────────┐
           │    RETURN    │
           └──────────────┘
```

25

## FIG. 10A

| $\alpha_x$ | FIRST EIGEN VECTOR | SECOND EIGEN VECTOR |
|---|---|---|
| SAMPLE 1 | −0.03315147 | 0.004524849 |
| SAMPLE 2 | −0.03315147 | 0.004524849 |
| SAMPLE 3 | −0.03315147 | 0.004524849 |
| SAMPLE 4 | 0.00703693 | −0.031475913 |
| SAMPLE 5 | 0.00703693 | −0.031475913 |
| SAMPLE 6 | 0.00703693 | −0.031475913 |
| SAMPLE 7 | 0.02611787 | 0.026941463 |
| SAMPLE 8 | 0.02611787 | 0.026941463 |
| SAMPLE 9 | 0.02611787 | 0.026941463 |

## FIG. 10B

| $w_y$ | FIRST EIGEN VECTOR | SECOND EIGEN VECTOR |
|---|---|---|
| FIRST GROUP (WILD RABBITS) | −0.7550584 | −0.1149792 |
| SECOND GROUP (WHHL RABBITS; WITH DOSING) | 0.1352623 | 0.561282 |
| THIRD GROUP (WHHL RABBITS; WITHOUT DOSING) | 0.6197961 | −0.4463028 |

## FIG. 10C

| $t$ | FIRST COMPONENT | SECOND COMPONENT |
|---|---|---|
| SAMPLE 1 | −4.866 | 1.536 |
| SAMPLE 2 | −8.863 | 3.192 |
| SAMPLE 3 | −5.521 | 1.559 |
| SAMPLE 4 | 3.766 | −7.299 |
| SAMPLE 5 | 1.374 | −6.703 |
| SAMPLE 6 | 2.246 | −5.530 |
| SAMPLE 7 | 3.656 | 3.664 |
| SAMPLE 8 | 4.233 | 3.389 |
| SAMPLE 9 | 3.975 | 6.191 |

## FIG. 11A

La

| METABOLITES OF LIVER | CORRELATION COEFFICIENT | p-VALUE |
|---|---|---|
| Betaine | 0.666 | 0.050 |
| N,N-Dimethylglycine | 0.712 | 0.032 |
| Urate | 0.594 | 0.091 |
| Hypoxanthine | −0.849 | 0.004 |
| Inosine | −0.833 | 0.005 |
| Adenosine | −0.708 | 0.033 |
| Adenine | −0.670 | 0.048 |
| ⋮ | ⋮ | ⋮ |

## FIG. 11B

Lb

| METABOLITES OF HEART | CORRELATION COEFFICIENT | p-VALUE |
|---|---|---|
| Betaine | 0.758 | 0.018 |
| N,N-Dimethylglycine | 0.754 | 0.019 |
| Citrulline | −0.925 | 0.000 |
| ⋮ | ⋮ | ⋮ |

## FIG. 11C

Lc

| METABOLITES OF BRAIN | CORRELATION COEFFICIENT | p-VALUE |
|---|---|---|
| N5-Ethylglutamine | −0.723 | 0.028 |
| Citrulline | −0.804 | 0.009 |
| ⋮ | ⋮ | ⋮ |

## FIG. 11D

Ld

| METABOLITES OF PLASMA | CORRELATION COEFFICIENT | p-VALUE |
|---|---|---|
| N5-Ethylglutamine | −0.670 | 0.048 |
| ⋮ | ⋮ | ⋮ |

## FIG. 12A

KERNEL PLS
($\kappa = 0$)

SECOND COMPONENT (SCORE)

WHHL

WILD

WHHL +DOSING

FIRST COMPONENT (SCORE)

PENALTY OF GROUP ORDER

## FIG. 12B

KERNEL PLS-ROG
($\kappa = 0.5$)

SECOND COMPONENT (SCORE)

WHHL

WILD

WHHL +DOSING

FIRST COMPONENT (SCORE)

## FIG. 13A

| LIVER | KERNEL PLS | | | KERNEL PLS-ROG | | |
|---|---|---|---|---|---|---|
| | CORRELATION COEFFICIENT | p-VALUE | | CORRELATION COEFFICIENT | p-VALUE | |
| Betaine | 0.149 | 0.702 | | 0.666 | 0.050 | * |
| N,N-Dimethylglycine | 0.158 | 0.684 | | 0.712 | 0.032 | * |
| Urate | 0.060 | 0.878 | | 0.594 | 0.091 | |
| Hypoxanthine | −0.392 | 0.297 | | −0.849 | 0.004 | * |
| Inosine | −0.371 | 0.326 | | −0.833 | 0.005 | * |
| Adenosine | −0.243 | 0.529 | | −0.708 | 0.033 | * |
| Adenine | −0.451 | 0.223 | | −0.670 | 0.048 | * |

* : p<0.05

## FIG. 13B

| HEART | KERNEL PLS | | | KERNEL PLS-ROG | | |
|---|---|---|---|---|---|---|
| | CORRELATION COEFFICIENT | p-VALUE | | CORRELATION COEFFICIENT | p-VALUE | |
| Betaine | 0.423 | 0.256 | | 0.758 | 0.018 | * |
| N,N-Dimethylglycine | 0.186 | 0.632 | | 0.754 | 0.019 | * |
| Citrulline | −0.740 | 0.023 | * | −0.925 | 0.000 | * |

* : p<0.05

## FIG. 13C

| BRAIN | KERNEL PLS | | | KERNEL PLS-ROG | | |
|---|---|---|---|---|---|---|
| | CORRELATION COEFFICIENT | p-VALUE | | CORRELATION COEFFICIENT | p-VALUE | |
| N5-Ethylglutamine | −0.270 | 0.483 | | −0.723 | 0.028 | * |
| Citrulline | −0.470 | 0.202 | | −0.804 | 0.009 | * |

* : p<0.05

## FIG. 13D

| PLASMA | KERNEL PLS | | | KERNEL PLS-ROG | | |
|---|---|---|---|---|---|---|
| | CORRELATION COEFFICIENT | p-VALUE | | CORRELATION COEFFICIENT | p-VALUE | |
| N5-Ethylglutamine | −0.495 | 0.176 | | −0.670 | 0.048 | * |

* : p<0.05

## FIG. 13E

POSITIVE CORRELATION

NEGATIVE CORRELATION-

METABOLITE 1
SCORE
METABOLITE 2

SAMPLE NUMBER
1 2 3 4 5 6 7 8 9

FIG. 14A KERNEL PLS: METAGENOME DATA

FIG. 14B KERNEL PLS: METABOLOME DATA

FIG. 14C

KERNEL PLS: METAGENOME DATA + METABOLOME DATA

**FIG. 15A**

KERNEL PLS-ROG:
METAGENOME DATA

SECOND COMPONENT (SCORE)

FIRST COMPONENT (SCORE)

0th WEEK | 2nd WEEK | 4th WEEK | 6th WEEK | 10th WEEK | 12th WEEK | 16th WEEK

**FIG. 15B**

KERNEL PLS-ROG:
METABOLOME DATA

SECOND COMPONENT (SCORE)

FIRST COMPONENT (SCORE)

0th WEEK | 2nd WEEK | 4th WEEK | 6th WEEK | 10th WEEK | 12th WEEK | 16th WEEK

0th WEEK
2nd WEEK
4th WEEK
6th WEEK
10th WEEK
12th WEEK
16th WEEK

**FIG. 15C**

KERNEL PLS-ROG:
METAGENOME DATA
+ METABOLOME DATA

SECOND COMPONENT (SCORE)

FIRST COMPONENT (SCORE)

0th WEEK | 2nd WEEK | 4th WEEK | 6th WEEK | 10th WEEK | 12th WEEK | 16th WEEK

## INTERNATIONAL SEARCH REPORT

| | International application No. |
|---|---|
| | PCT/JP2016/084509 |

| A. CLASSIFICATION OF SUBJECT MATTER |
|---|
| *G06N99/00*(2010.01)i, *G06F17/15*(2006.01)i, *G06F19/24*(2011.01)i |

According to International Patent Classification (IPC) or to both national classification and IPC

| B. FIELDS SEARCHED |
|---|
| Minimum documentation searched (classification system followed by classification symbols) |
| G06N99/00, G06F17/15, G06F19/24, G01N33/48, G01N33/50 |

Documentation searched other than minimum documentation to the extent that such documents are included in the fields searched

| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | US 2015/0276764 A1 (SLUPSKY Carolyn), 01 October 2015 (01.10.2015), entire text; all drawings & WO 2014/071411 A1 | 1–11 |
| E,A | JP 2016-200435 A (University of Yamanashi), 01 December 2016 (01.12.2016), entire text; all drawings (Family: none) | 1–11 |

☐ Further documents are listed in the continuation of Box C. ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 07 February 2017 (07.02.17) | 21 February 2017 (21.02.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030200040 A

**Non-patent literature cited in the description**

- **T. OOGA et al.** Metabolomic anatomy of an animal model revealing homeostatic imbalances in dyslipidaemia. *Mol. Biosyst,* vol. 7 (4
- **H. YAMAMOTO.** PLS-ROG: Partial least squares with rank order of groups. *COBRA Preprint Series, Working Paper 100,* October 2012
- **C. URBANIAK et al.** Effect of chemotherapy on the microbiota and metabolome of human milk. *Microbiome,* 2014
- **LOZUPONE C et al.** UniFrac: a new phylogenetic method for comparing microbial communities. *Appl Environ Microbiol,* 2005